# EUROPEAN PATENT APPLICATION

(11) **EP 2 562 666 A2**
(43) Date of publication of application: **27.02.2013**
(21) Application number: 12169325.3
(22) Date of filing: 24.05.2012
(51) Int. Cl.: G06F 19/00

(54) **Fitness equipment subscription system**

(30) Priority: 24.08.2011 US 201113216489
(71) Applicant: Precor Incorporated, Woodinville, WA 98072 (US)
(72) Inventor: Barker, Robert, Camberley, Surrey GU16 7ER (GB)
(74) Representative: Docherty, Andrew John

(57) **Abstract**

A method and apparatus receive an identification of a user at a fitness equipment unit, retrieve a current subscription plan for the user based upon the identification and make features for the fitness equipment unit in the current subscription plan of the user available for use by the user on the fitness equipment unit.

## Description

### FIELD

The present invention relates to a fitness equipment subscription system and method.

### BACKGROUND

In health and fitness facilities, members are often given the same service offer for using the facilities and equipment. In other words, all members are provided with the same access to the same features on the various fitness equipment in the facility. As a result, such health and fitness facilities cannot economically offer services to satisfy both (1) members on a budget with low experience demands and (2) more affluent members with high experience demands.

### SUMMARY

A fitness equipment subscription system may comprise:
a fitness equipment unit operable in a base mode with one or more selectable optional supplemental features;
one or more inputs associated with the fitness equipment unit that receives input identifying a user using the fitness equipment unit; and
a controller in communication with the input and the fitness equipment unit, wherein the controller is configured to make available the one or more optional features based upon the identity of the user and a subscription plan associated with the user.

A fitness equipment subscription system may comprise:
a fitness equipment unit operable in a base mode with one or more selectable optional supplemental features;
one or more inputs associated with the fitness equipment unit that receives input identifying a user using the fitness equipment unit; and
a controller in communication with the input and the fitness equipment unit, wherein the controller is configured to limit use of the fitness equipment unit based on a subscription plan associated with the user.

A fitness equipment subscription system may comprise:
a fitness equipment unit operable in a base mode with one or more selectable optional supplemental features;
one or more inputs associated with the fitness equipment unit that receives input identifying a user using the fitness equipment unit; and
a controller in communication with the input and the fitness equipment unit, wherein the controller is configured to at least one of make available the one or more optional features based upon the identity of the user and a subscription plan associated with the user, and limit use of the fitness equipment unit based on a subscription plan associated with the user.

The subscription plan may be purchased by the user. The subscription plan may be provided to the user.

The subscription plan associated with the user may identify a quantity of use of the fitness equipment unit available to the user.

The subscription plan associated with user may identify a universal time period available to the user for using the fitness equipment unit.

The fitness equipment unit may include a display. The controller may generate control signals causing the one or more selectable optional features to be presented on the display. The base mode may comprise a first set of visual presentations on the display. The one or more optional supplemental features may comprise a second set of visual presentations on the display.

The controller may be configured to present a paid subscription status of the user that identifies purchases of optional supplemental features.

The controller may be configured to cause the display to present offers for additional supplemental features not currently in the subscription plan of the user.

The one or more inputs may be configured to receive payment authorization from the user for the purchase of additional supplemental features.

The base mode may comprise a first set of one or more exercise motions and wherein the one or more optional supplemental features comprises a second set of one or more exercise paths of motion.

The at least one input may be configured to receive a selection of a feature contained in the subscription plan of the user.

The fitness equipment unit may define a first fitness equipment unit and the system may comprise a second fitness equipment unit.

A second input may be associated with the second fitness equipment unit.

The controller may be in communication with the second fitness equipment unit.

The second fitness equipment unit may be operable in the base mode and the one or more selectable optional supplemental features.

The controller may be configured to make available the one or more optional features on the second fitness equipment unit based upon an identity of a second user and a second subscription plan associated with the second user.

The first fitness equipment unit may be operable in a first base mode and one or more first selectable optional supplemental features, and the second fitness equipment unit may be operable in a second base mode different than the first base mode and one or more second selectable optional supplemental features.

The controller may be configured to make available the one or more second optional features on the second fitness equipment unit based upon an identity of a second user and a second subscription plan associated with the second user.

The one or more optional features may comprise forwarding at least a media resumption point from the fitness equipment unit to an external media player.

A method may comprise:
receiving an identification of a user at a fitness equipment unit;
retrieving a current subscription plan for the user based upon the identification;
making features for the fitness equipment unit in the current subscription plan of the user available for use by the user on the fitness equipment unit.

A method may comprise:
receiving an identification of a user at a fitness equipment unit;
retrieved a current subscription plan for the user based upon the received identification of the user at the fitness equipment unit from a database of a plurality of subscription plans for a plurality of potential users of the fitness equipment unit;
making features for the fitness equipment unit in the current subscription plan of the user available for use by the user on the fitness equipment.

The current subscription plan may be retrieved from a database of a plurality of subscription plans for a plurality of potential users of the fitness equipment unit.

The subscription plan may comprise features for a plurality of different fitness equipment units for each of the plurality of potential users.

The methods defined above may comprise:
making features in the current subscription plan available for selection by the user at the fitness equipment unit;
receiving a selection of one or more of the features in the current description plan for the user;
controlling the fitness equipment unit to use the one or more features based on the selection.

The methods defined above may comprise receiving at the fitness equipment unit from the user selections of one or more features to add to the current subscription plan for the user.

The methods defined above may comprise receiving at the fitness equipment unit from the user payment for the one or more features to add to the current subscription plan for the user.

The base mode may comprise a first set of one or more exercise motions and wherein the one or more optional supplemental features comprises a second set of one or more exercise paths of motion.

The fitness equipment unit may include a display, wherein the base mode comprises a first set of visual presentations on the display and wherein the one or more optional supplemental features comprise a second set of visual presentations on the display.

The methods defined above may comprise presenting offers for an optional purchase of additional supplemental features not currently in the subscription plan of the user on a display associated with the fitness equipment unit.

The current subscription plan may include a first feature having a first use quantity and a second feature having a second use quantity different than the first use quantity.

### BRIEF DESCRIPTION OF THE DRAWINGS

These and other features and aspects of the present invention will now be described, by way of example only, with reference to the accompanying drawings, in which:
Figure 1 is a schematic illustration of a fitness equipment subscription system according to an example embodiment;
Figure 2 is a plan view of an example visual presentation of a current subscription plan and upgrade offer of the system of Figure 1;
Figure 3 is a plan view of another example visual presentation of a current subscription plan and upgrade offer of the system of Figure 1;
Figure 4 is a top left perspective view of a fitness equipment unit according to an example embodiment with portions schematically shown;
Figure 4A is a top right perspective view of the fitness equipment unit of Figure 4;
Figure 5 is another top perspective view of a portion of the fitness equipment unit of Figure 4;
Figure 6 is another top perspective view of a portion of the fitness equipment unit of Figure 4;
Figure 7 is another top perspective view of a portion of the fitness equipment unit of Figure 4;
Figure 8 is a right side elevational view of the fitness equipment unit of Figure 4;
Figure 9 is a partial rear elevational view of a portion of the fitness equipment unit of Figure 4;
Figure 10 is a rear elevational view of a portion of the fitness equipment unit of Figure 17;
Figure 11A is a diagram illustrating flexible elements of the fitness equipment unit of Figure 4 at one step height setting;
Figure 11 B is a diagram illustrating flexible elements of the fitness equipment unit of Figure 4 at another step height setting;
Figure 12 is a front perspective view of another embodiment of the fitness equipment unit of Figure 4;
Figure 13 is a front perspective view of the fitness equipment unit of Figure 4 with portions omitted for purposes of illustration;

### DETAILED DESCRIPTION OF THE EXAMPLE EMBODIMENTS

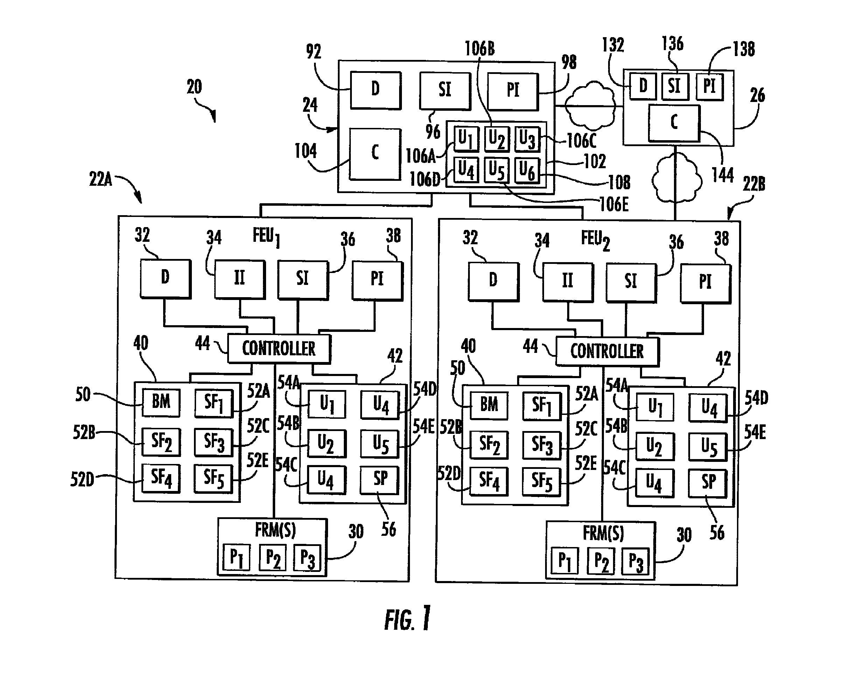

Figure 1 schematically illustrates fitness equipment subscription system 20 according to an example embodiment. Fitness equipment subscription system 20 is configured to provide different access to or different features for an exercise machine or fitness equipment unit to different users or members at a gym or fitness facility depending upon the individual member's subscription plan. Fitness equipment subscription system 20 enables those members on a budget to subscribe to lower-cost subscription options while also enabling more affluent members, or members who desire more options, to subscribe to higher-cost subscription options with a greater number of features. As a result, gyms, health clubs or other fitness facilities may customize their offerings to accommodate a wide range of needs and budgets.

Fitness equipment subscription system 20 comprises exercise machines or fitness equipment units 22A, 22B (collectively referred to as fitness equipment units 22), remote subscription manager 24 and remote subscription monitor 26. Each of fitness equipment units 22 comprises an individual fitness or exercise machine or piece of equipment configured to receive and resist force from a person exercising. Examples of fitness equipment units include, but are not limited to, elliptical machines, stair steppers or climbers, treadmills, adaptive motion machines, rowing machines, bench press machines, upright and recumbent cycles, cross trainers, strength training equipment and the like. In the example illustrated, fitness equipment units 22A and 22B comprise different types of fitness equipment units configured to exercise different muscles of a person. For example, in one embodiment, fitness unit 22A may comprise an adaptive motion machine while fitness equipment unit 22B comprises one of elliptical machine, a treadmill or other exercise equipment.

In another embodiment, fitness equipment units 22A and 22B may both comprise the same type of fitness equipment unit configured exercise generally the same muscles of a person exercising, but where fitness equipment units 22A and 22B are made by different manufacturers, have different available features, are different models, are of a different age or usage, or are at different locations within the same exercise facility (e.g., one of fitness equipment units 22 being located next to a window, a refreshment stand or a certain piece of exercise equipment while the other of fitness equipment units 22 being located next to a locker room, a water cooler or other amenity). According to one embodiment, fitness equipment units 22A and 22B are both located at the same fitness facility. In other embodiments, fitness community 22A and 22B may be located at distinct locations, such as distinct fitness facilities.

Each of fitness equipment units 22 comprise one or more force receiving members 30, display 32, identity input 34, selector input 36, payment input 38, control instructions 40, subscription database 42 and a controller 44. Force receiving members 30 comprise movable members or structures configured to be contacted by an anatomy of a person exercising and to receive force from the anatomy, wherein the force receiving members may, in turn, exert a resistance against movement and against the force received from the anatomy of the person exercising. Examples of force receiving members 30, include, but are not limited to, foot pedals or foot supports in an elliptical machine, an adaptive motion machine or rowing machine; a belt of a treadmill; or a handle or grip of an elliptical, and adaptive motion machine, rowing machine other device which exercise the upper body of a person exercising.

As schematically illustrated in Figure 1, in the example illustrated, each of force receiving members 30 is movable through one of a plurality of possible different paths or different ranges of movement or motion. For example, in one embodiment wherein one or both of fitness equipment units 22 comprise an adaptive motion machine, force receiving members 30 may be selectively movable through a more vertical path P1 (similar to stair stepping), a more horizontal path P2 (similar to an elliptical machine, strider or step machine) and a hybrid path P3. In each of such paths P1-P3, one of the movable members 30 may have the same X coordinate position in space but a different Y or Z coordinate position in space, or vice versa. In other embodiments, force receiving members 30 may not offer different selectable paths or ranges of motion.

Display 32 comprises a monitor, screen or other device configured to present visual information to a user of the associated fitness community 22 while the user is exercising. For example, display 32 may comprise an LCD screen. In another embodiment, display 32 may comprise an array or series of individual lights or light emitting diodes that are selectively illuminated to provide visual information. In one embodiment, display 32 may be a part of a touch screen which may also serve as one or more of identity input 34, selector input 36 or payment input 38.

In one embodiment, display 32 is fixedly mounted to a frame of the associated fitness equipment unit and supported such that a person may view display 32 when exercising. In yet another embodiment, display 32 may be provided by a portable device which is removably connectable to the associated fitness equipment unit 22. For example, display 32 may be provided by a hand held personal data device such as a personal digital assistance (PDA), portable media player (such as an IPOD), MP3 player or similar portable device having a display which is connected to controller 44 via a plug-in or port or wirelessly, wherein the portable device is supported by the frame during such exercise or is held by the user exercising.

Identity input 34 comprises a device (and associated firmware and software) associated with fitness equipment unit 22 that is configured to receive input identifying a particular human user using fitness equipment unit 22. In one embodiment, input 34 may be associated with fitness equipment unit 22 by being directly connected to, mounted upon or provided as part of fitness equipment unit 22A and 22B. In another embodiment, input 34 may be associated with fitness equipment unit 22A or 22B by being located within a particular area or region of a facility containing the associated fitness equipment unit 22. For example, input 24 may be provided on a wall proximate to the associated fitness equipment unit 22.

According to one embodiment, input 34 comprises a user interaction device that actively requests, prompts or receives input directly from the person using the associated fitness of unit 22. In such embodiments, input 34 may comprise a keyboard or keypad, a touch screen, a microphone with a computing device having associated voice or speech recognition software, a card reader or a biometric sensing device, wherein the person using fitness equipment unit 34 enters his or her identification information either manually, audibly or by positioning a card (magnetic or electronic) in a card reader or by positioning his or her anatomy with respect to the biometric sensing device.

In yet other embodiments, input 34 may comprise a passive person identification device which identifies a person using the associated fitness equipment unit 22 without any active participation or specific actions on part of the person using fitness equipment unit 22. For example, input 34 may comprise a camera or other image capturing device and a computing device having associated face recognition software. Input 34 may comprise a sensing device that senses a security or identification token, sometimes referred to as a key fob, assigned or designated to the particular user or person and located within a predetermined proximity to the sensing device, such as when the person is seated upon a portion of the fitness equipment unit or is using the fitness equipment unit. In some embodiments, such identification tokens may rely on radio frequency identification tags and transponders. Overall, input device 34 provides controller 44 with the identity of the particular person using or about to use the associated fitness equipment unit 22.

Selector input 36 comprises a device or mechanism by which a person exercising may enter or input selections to controller 44. In particular, selector input 36 is configured such that a person may choose or select one or more optional features for use during exercise with the associated fitness equipment unit 22, depending upon the person's subscription plan. In some embodiments, selector input 36 may also be configured to allow a person exercising to upgrade his or her subscription plan while at the associated fitness equipment unit 22. Examples of selector input 36 include, but are not limited to, components of a touch screen incorporated as part of display 32 or another display, a touchpad, a mouse, stylus, a microphone with speech recognition software, keyboard or keypad.

Payment input 38 comprises a device or mechanism located at each of fitness community 22A and 22B by which a person may authorize or remit payment when subscribing to a new or different subscription plan or when upgrading the current subscription plan. In one embodiment, payment input 38 may comprise a sensing device (and associated firmware and software) that senses a payment authorization token, sometimes referred to as a key fob, assigned or designated to the particular user or person and located within a predetermined proximity to the sensing device, such as when the person is seated upon a portion of the fitness equipment unit or is using the fitness equipment unit. In some embodiments, such payment authorization tokens may rely on radio frequency identification tags and transponders. In another embodiment, payment input 38 may comprise a card reader, such as a magnetic card reader (configured to read a swiped credit card, debit card, membership card or the like). In yet other embodiments, payment input 38 may comprise the keyboard, keypad or other input device (and associated firmware and software) that allows the person enter his or her credit card information authorizing payment. As such, in some embodiments, payment input 38 may be provided as part of one or more of display 32, identification input 34 and/or selector input 36. In some embodiments, payment input 38 may be omitted and not included as part of a fitness equipment unit.

Payment through payment input 38 or other means outside of payment input 38 may be in the form of any of multiple "currencies". Payment may be the remission of "currency" comprising monetary forms, i.e. US dollars etc. Payment may also comprise the expenditure or redemption of non-monetary forms of "currency" or credit such as reward points. In one embodiment, such as reward points may be awarded to persons or earned by persons in one of multiple avenues. For example, a person may be awarded points or credit that may be redeemed for the purchase of a subscription plan as part of a promotion such as when a person first signs up for a program or joins a fitness facility. A person may be awarded points or credit when a person makes a voluntary contribution to a charity or food drive through the fitness or health club. These are but a few examples of promotions by which such credits or points may be awarded.

A person may earn such points or credit from a fitness facility or health club based upon health or fitness related standards or goals being satisfied or met by the person or a group of persons. For example, a person may be rewarded with points or credit based upon the the person or group of persons attendance record at the fitness facility or based upon the number of hours spent by the person or group of persons at the fitness facility or spent by the person or group of persons on a particular exercise machine or a particular exercise regimen. A person may be rewarded with points or credit based upon a person or group of persons satisfying a particular fitness goal or objective such as a target heart rate, a number of miles, or other fitness or health metrics. Such rewards may be awarded by a fitness facility or larger health club, wherein an outside third party pays the fitness facility for such rewarded credit or points. For example, a health insurance company may partner with a fitness facility to reward a person's or a group of person's attendance or fitness achievements.

Control instructions 40 comprise instructions or control logic embodied as firmware or software in one or more memory structures. Control instructions 40 provide instructions for controller 44. Control instructions 40 comprise different blocks, modules or sets of control programming or routines for the operation of the associated fitness equipment unit 22A, 22B. In the example illustrated, control instructions 40 comprises base mode instructions 50 and optional or supplemental feature instructions 52A, 52B, 52C, 52D, 52E (collectively referred to as supplemental feature instructions 52).

Base mode instructions 50 comprise computer readable programming or instructions for controller 44 so as to control the operation of fitness equipment units 22 in a default or baseline mode of operation. The baseline mode of operation is a minimum or default operation characteristics for the particular fitness equipment unit 22: the minimum number of paths or ranges of motion, the minimum visual presentations to be presented on display 32 and generally the minimum overall user experience. In one embodiment, absent a subscription plan with any additional supplemental features, controller 44 operates fitness equipment unit 22A or 22B in the base mode. In another embodiment, a baseline subscription plan may include the base mode of operation operating under base mode 50 instructions but not any additional supplemental features which would be provided by supplemental feature instructions 52.

Supplemental feature instructions 52 comprise computer readable programming or instructions so as to control the operation of fitness equipment units 22 with one or more additional supplemental features. Such supplemental features may either replace the base mode features with enhanced features or may add to or build upon the features provided by the base mode of operation. According to one embodiment, such supplemental features are purchasable by the health fitness facility member or user as part of a prescription plan upgrade. The supplemental features may upgrade the operation of display 32, increasing the available content or interactive features or the overall visual presentation quantity presented by controller 44 on display 32 or may upgrade the operation of force receiving members 30, providing a greater range of motion, additional paths, additional or alternative resistance options and the like.

Subscription database 42 comprises a database of current subscription plans 54A, 54B, 54C, 54D, 54E (collectively referred to as subscription plans 54) for different potential users of the associated fitness equipment unit 22. In one embodiment, subscription database 42 may additionally include a database of different available subscription plans or features that may be purchased along with their associated prices (schematically represented by subscription plan 56). Each subscription plan may include access to or make available the base mode and one or more supplemental features as provided by base mode instruction 50 and supplemental feature instructions 52 under the control of controller 44.

In one embodiment, each subscription plan 54, 56 may additionally include a pre-purchased quantity relating to the use of fitness equipment unit 22A, 22B. For example, each subscription plan 54, 56 may additionally include a prepurchased number of hours that the subscriber may use a particular fitness equipment unit 22A, 22b. The hours quantity may be a total number of hours which is gradually consumed by a subscriber during use of the fitness pivot unit or may be a total number of hours during a predefined time period (e.g., 5 hours per week; 15 hours per month; 5 hours per calendar week; 15 hours per calendar year).

In another embodiment, the prepurchased quantity may be defined in terms of a metric on the fitness equipment unit itself. For example, each subscription plan 54, 56 may include a prepurchased number of miles such as when the fitness equipment unit 22A or 22B is a treadmill, elliptical machine or adaptive motion machine. In another embodiment, the subscription plan 54, 56 may include a prepurchased number of pounds such as when the fitness equipment unit 22A, 22B involves the listing of weights. In yet other embodiments, the subscription plan 54, 56 may include a pre-purchased quantity relating to wear of the fitness equipment unit. In such a manner, the cost of prescription plan could be directly tied to maintenance, repair or replacement of the fitness equipment unit being used.

In yet another embodiment, the subscription plan 54, 56 may include a pre-purchased or predefined universal time period or range for which the particular fitness equipment unit 22A, 22B is available for use. For example, the subscription plan may provide user with access to the particular fitness equipment unit on particular days of the week, such as Monday, Wednesday and Friday, only on weekends, during particular hours of each day (8 AM to 11 AM), or during particular hours on selected days (8 AM to 11 AM on Monday Wednesday and Friday, 2 PM to 3 PM on Saturday and 8 PM to 10 PM on Sunday). With such subscription plans 54, 56, prime times or more busy times for a particular fitness equipment unit 22A, 22B may have a higher subscription price while less prime times or less busy times for a particular fitness equipment unit 22A, 22B may be provided with a lower subscription price. As a result, available time for use of a fitness equipment unit 22A, 22B may be allotted at different prices to different users to accommodate different budgets or price points for different users while the same time maximizing income from the fitness equipment unit 22A, 22B.

Likewise, in facilities where identical fitness equipment units are located at different locations within the facility, subscription plans 54, 56 for each of the identical equipment units may have different prices for an otherwise identical subscription plan depending upon demand for a particular fitness equipment unit at a particular location within the fitness facility (more convenient versus less convenient location) or within close proximity to a desired amenity (locker room, refreshment station or other fitness equipment units).

Subscription database 42 is stored in a memory structure embodied in fitness equipment unit 22A, 22B. In other embodiments where similar subscription databases are maintained elsewhere, such as subscription manager 24, subscription database 42 may be omitted as part of the particular fitness equipment unit 22A, 22B. although each of the description database 42 is illustrated as including or storing current subscription plans for six users, U1-U6, subscription plans may be enlarged or reduced in size in other embodiments.

Controller 44 comprise one or more processing units provided as part of fitness equipment unit 22A, 22B configured to follow instructions, contained in control instructions 40 and based in part upon the current subscription plan for the current member exercising on the particular fitness equipment unit 22A, 22B, to control the operation of force receiving members 30 and display 32. For purposes of this application, the term "processing unit" shall mean a presently developed or future developed processing unit that executes sequences of instructions contained in a memory. Execution of the sequences of instructions causes the processing unit to perform steps such as generating control signals. The instructions may be loaded in a random access memory (RAM) for execution by the processing unit from a read only memory (ROM), a mass storage device, or some other persistent storage. In other embodiments, hard wired circuitry may be used in place of or in combination with software instructions to implement the functions described. For example, controller 44 may be embodied as part of one or more application-specific integrated circuits (ASICs). Unless otherwise specifically noted, the controller is not limited to any specific combination of hardware circuitry and software, nor to any particular source for the instructions executed by the processing unit.

In operation according to one embodiment, controller 44 awaits receipt of input or information from identification input 34 identifying a member of the fitness facility or person requesting to use a particular fitness equipment unit 22A, 22B. Upon receipt of the identification input, controller 44 accesses subscription database 42 and retrieves the current or active subscription plan 54 associated with the particular user. In some embodiments, a "user" may comprise an individual person. In other embodiments, a "user" may comprise an organization or a collective group of individuals who may have purchased a common subscription plan together as a group possibly at a reduced price.

Once the appropriate subscription plan for the particular user that was identified by the identifying input received from identification input 34 has been retrieved by controller 44, controller 44 generate control signals causing display 32 to present a paid subscription status of the user that identifies those features or options currently available in the current subscription plan. Controller 44 follows by awaiting input from selector input 36 identifying which of the available supplemental features that the individual exercising wishes to use during the exercise session. Upon receiving the individuals selection, controller 44 accesses the associated control instructions 50 and/or 52 containing instructions 40. Controller 44 generate control signals controlling one or both of display 32 and force receiving members 30 based upon the selection.

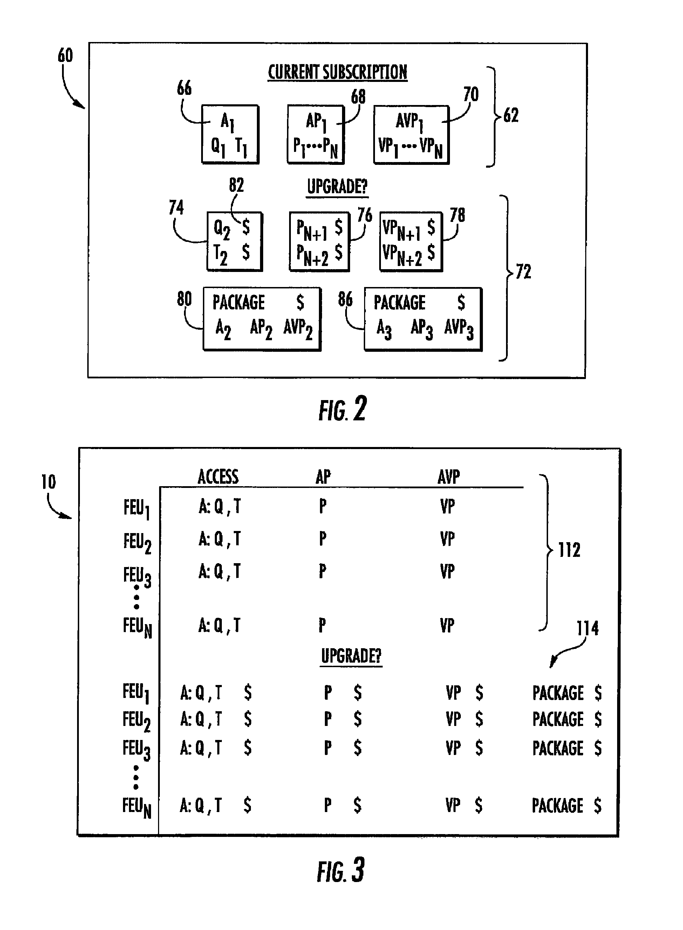

Figure 2 illustrates one example display presentation 60 that may be presented by controller 44 on display 32 based upon the identification of the person exercising and his or her current subscription plan as retrieved from subscription database 42. As shown by Figure 2, presentation 60 identifies the current subscription plan 62 for the particular fitness equipment unit 22A or 22B at which the user is stationed. In the example illustrated, the current subscription plan 62 includes current accessibility 66 (A), available parameters 68 (AP) and available video or visual presentations 70 (AVP). The current accessibility 66 identifies any limitations on the person's use of the particular fitness equipment unit 22A, 22B, such as a quantity limitation Q (e.g., hours, days, miles wear) or a universal time limitation T (particular days of the week, such as Monday, Wednesday and Friday, only on weekends, during particular hours of each day (8 AM to 11 AM), or during particular hours on selected days (8 AM to 11 AM on Monday Wednesday and Friday, 2 PM to 3 PM on Saturday and 8 PM to 10 PM on Sunday)). In some embodiments, accessibility may not be limited or may not be part of a subscription plan.

Available parameters (AP) 68 comprises various parameters directly pertaining to the movement of force receiving members 30. Available parameters 68 may include one or more optional or selectable parameters (P1 to PN) such as one or more paths of motion (such as with an adaptive motion machine) one or more ranges of motion, and/or one or more ranges or levels of resistance against the movement of force receiving members 30. In some embodiments, the available parameters may not be a selectable feature or may not be part of a subscription plan.

Available visual presentations (AVP) 70 comprise various video content, video features or video quality choices (including audio) (VP1 to VPN) for a person exercising. In one embodiment, the current subscription plan for the person exercising may be provide library of different content that the person exercising may choose from during exercise. Examples of such content include, but are not limited to, movies, television programming, music, or other audio or visual content. Such content subscriptions may be individual media choices or may comprise packages of multiple media choices. For example, one subscription may just include major network television stations whereas another subscription additionally includes premium channels such as premium movie channels (HBO, Showtime) or ESPN. In one embodiment, one subscription plan may include Internet access or Internet surfing. In one embodiment, the subscription plan for the person exercising may include different levels of quality for content, such as high-definition, low definition, three-dimensional and the like. In some embodiments, the available visual presentation 70 in a subscription plan may provide one or more interactive features such as games, simulations, exercise challenges, exercise trainer assistance, or inter or intra-facility exercise events or competitions, wherein sensed movement of force receiving members 30 is utilized by controller 44 as an input to the game, simulation, challenge, trainer assistance or competition which is presented on display 32.

In one embodiment, the available visible presentation of a particular subscription plan 54, 56 may comprise a portability feature, wherein controller 44 (or controller 104) records the point or location in the media presentation where the presentation was interrupted or temporarily stopped (media resumption point) such that when the person exercises on FEU 22A or on another exercise device, such as FEU 22B, at a later time, the person or display of the other exercise device or machine may automatically resume the same media presentation at the same point. For example, a subscription plan may allow a person to watch a movie on Monday while exercising on a treadmill and to resume watching the same movie from the exact point in the movie where the person left off on Monday while exercising on an elliptical machine on Tuesday.

In one embodiment, the subscription plan may provide the subscriber with the additional opportunity to have controller 44, controller 104 or controller 144 forward the media with the media resumption point (or just the media resumption point) to another media controller or media display location as designated by the subscriber, allowing the subscriber to resume watching the media at the other media controller or media display location (at home, at another location in the same home, building or facility in which the exercise machine is located, on his or her portable media player (IPHONE, IPAD, IPOD, personal data assistant, etc.)) at a later time from the exact location in the media presentation where he or she left off after finishing exercising. Such forwarding of the media and media resumption point or just the media resumption point may be achieved by transmission across the internet or across a local area network (LAN), by the transferal to a portable memory carried by the subscriber (i.e. a flash drive, thumb drive, memory card or the like), or by direct transmission to a portable media player temporarily connected to the controller 44 or controller 104 by a USB connection, wired connection, infrared or Bluetooth connection etc.).

The presentation of the current subscription plan 62 for the person exercising allows the person to see his or her current options and to possibly select one or more of the options using selector input 36.

According to one embodiment, controller 44 may further generate control signals causing display 32 to present various upgrade options or offers 72. As shown by Figure 2, the upgrade offers may include offers to upgrade accessibility 74 to the particular fitness equipment unit 22A, 22B such as increasing either one or more quantity limitations Q or universal time limitations T. The upgrade offers may include one or more additional parameters (P) 76 or one or more additional visual presentations (VP) 78. In addition, the upgrade offers may include prepackaged sets 80 of upgrade options, wherein each set 80 includes a different group of accessibility, additional available parameters and additional available visual presentations. With each offer, presentation 60 displays a price ($) 82 for the upgrade. The cost or price of such upgrades may constitute a one-time payment, a monthly payment, yearly payment of the like.

If the user wishes to upgrade his or her subscription plan for the particular fitness equipment unit 22A, 22B while the person is physically at the fitness equipment unit 22A, 22B, person may select the offer using selector input 36 and may remit payment using payment input 38. In one embodiment, actual payment may not be required, wherein the person merely authorizes his or her account to be billed or charged. Once an upgrade is selected and paid or charged, controller 44 updates the person subscription plan 54 in subscription database 42.

At predetermined times, controller 44 further maintains subscription database 42 by comparing the current date or time with any expiration date for any feature in the subscription plan. Controller 44 culls or removes features from the subscription plan that have expired. In embodiments where subscriptions have a termination or expiration date, controller 44 may additionally generate control signals causing visual presentation 60 to additionally inform the user of features currently in the current subscription that are about to expire.

Subscription manager 24 comprises a central device in communication with each of a plurality of fitness equipment units in the same facility or in multiple different facilities. Such communication may be provided in a wired or wireless fashion. This communication may be across an intranet or the Internet. In other embodiments, subscription manager 24 may indirectly communicate with multiple fitness equipment units through use of data carrying structures, such as cards and the like, that are written upon and/or read from by manager 24 and multiple fitness equipment units 22, wherein the data carrying structures are manually carried between manager 24 and the various fitness equipment units 22.

In particular embodiments, subscription manager 24 facilitates the monitoring, oversight and adjustment of a subscription plan for a multitude of different fitness equipment units for a single person and such subscription plans from multiple individuals. Subscription manager 24 may be operated by a manager or owner of a fitness facility. The description manager 24 comprises display 92, selector input 96, payment input 98, subscription database 102 and controller 104.

Display 92 comprises a monitor, screen or other device configured to present visual information. For example, display 92 may comprise an LCD screen. In another embodiment, display 92 may comprise an array or series of individual lights or light emitting diodes that are selectively illuminated to provide visual information. In one embodiment, display 92 may be a part of a touch screen which may also serve as one or more of selector input 96 or payment input 98.

Selector input 96 comprises a device or mechanism by which a person exercising may enter or input selections to controller 104. In particular, selector input 96 is configured such that a person may choose or select one or more optional features for modifying the person's subscription plan. Examples of selector input 96 include, but are not limited to, components of a touch screen incorporated as part of display 92 or another display, a touchpad, a mouse, stylus, a microphone with speech recognition software, keyboard or keypad.

Payment input 98 comprises a device or mechanism remotely located from fitness equipment units 22 by which a person may authorize or remit payment when subscribing to a new or different subscription plan or when upgrading the current subscription plan. In one embodiment, payment input 98 may comprise a sensing device (and associated firmware and software) that senses a payment authorization token, sometimes referred to as a key fob, assigned or designated to the particular user or person and located within a predetermined proximity to the sensing device, such as when the person is seated upon a portion of the fitness equipment unit or is using the fitness equipment unit. In some embodiments, such payment authorization tokens may rely on radio frequency identification tags and transponders. In another embodiment, payment input 98 may comprise a card reader, such as a magnetic card reader (configured to read a swiped credit card, debit card, membership card or the like). In yet other embodiments, payment input 98 may comprise the keyboard, keypad or other input device (and associated firmware and software) that allows the person enter his or her credit card information authorizing payment. As such, in some embodiments, payment input 98 may be provided as part of one or more of display 92, and/or selector input 96. In some embodiments, payment input 98 may be omitted and not included as part of manager 24.

Subscription database 102 comprises a database of current subscription plans 106A, 106B, 106C, 106D, 106E (collectively referred to as subscription plans 106) for different potential users of the associated fitness equipment units 22A and 22B (as well as addition fitness equipment units 22 (not shown)). In one embodiment, subscription database 102 may additionally include a database of different available subscription plans or features that may be purchased along with their associated prices (schematically represented by subscription plan 108). Each subscription plan may include access to or make available the base mode and one or more supplemental features as provided by base mode instruction 50 and supplemental feature instructions 52 under the control of controller 44 for each of multiple fitness equipment units 22.

In one embodiment, each subscription plan 106, 108 may additionally include a pre-purchased quantity relating to the use of fitness equipment units 22A, 22B. For example, each subscription plan 106, 108 may additionally include a prepurchased number of hours that the subscriber may use a particular fitness equipment unit 22A, 22b. The hours quantity may be a total number of hours which is gradually consumed by a subscriber during use of the fitness pivot unit or may be a total number of hours during a predefined time period (e.g., 5 hours per week; 15 hours per month; 5 hours per calendar week; 15 hours per calendar year).

In another embodiment, the prepurchased quantity may be defined in terms of a metric on the fitness equipment unit itself. For example, each subscription plan 106, 108 may include a prepurchased number of miles such as when the fitness equipment unit 22A or 22B is a treadmill, elliptical machine or adaptive motion machine. In another embodiment, the subscription plan 106:08 may include a prepurchased number of pounds such as when the fitness equipment unit 22A, 22B involves the lifting of weights. In yet other embodiments, the subscription plan 106:08 may include a pre-purchased quantity relating to wear of the fitness equipment unit. In such a manner, the cost of prescription plan could be directly tied to maintenance, repair or replacement of the fitness equipment unit being used.

In yet another embodiment, the subscription plan 106, 108 may include a pre-purchased or predefined universal time period or range for which the particular fitness equipment unit 22A, 22B is available for use. For example, the subscription plan may provide user with access to the particular fitness equipment unit on particular days of the week, such as Monday, Wednesday and Friday, only on weekends, during particular hours of each day (8 AM to 11 AM), or during particular hours on selected days (8 AM to 11 AM on Monday Wednesday and Friday, 2 PM to 3 PM on Saturday and 8 PM to 10 PM on Sunday). With such subscription plans 106, 108, prime times or more busy times for a particular fitness equipment unit 22A, 22B may have a higher subscription price while less prime times or less busy times for a particular fitness equipment unit 22A, 22B may be provided with a lower subscription price. As a result, available time for use of a fitness equipment unit 22A, 22B may be allotted at different prices to different users to accommodate different budgets or price points for different users while the same time maximizing income from the fitness equipment unit 22A, 22B.

Likewise, in facilities where identical fitness equipment units are located at different locations within the facility, subscription plans 106, 108 for each of the identical equipment units may have different prices for an otherwise identical subscription plan depending upon demand for a particular fitness equipment unit at a particular location within the fitness facility (more convenient versus less convenient location) or within close proximity to a desired amenity (locker room, refreshment station or other fitness equipment units).

Controller 104 comprise one or more processing units provided as part of subscription manager 24 configured to follow instructions contained in a memory structure such as the same memory structure containing database 102. Controller 104 and the associated instructions contained in the memory structure are configured to facilitate communication between manager 24 and each of the fitness equipment units 22 being managed by manager 24. Such communication may be in a wired or wireless fashion (intranet, Internet). Controller 104 is further configured to revise or update subscription plans 106 in database 102 based upon either selections and purchases made at the individual fitness equipment unit 22 or selections and purchases made at manager 24 itself using display 92, selector input 96 and payment input 98.

In some embodiments, controller 104 may itself be configured to verify whether a particular user is authorized to select one or more supplemental features based upon the use identity as received by identity input 34 at the particular fitness equipment unit 22 and communicate to manager 24 and the particular user subscription plan stored in database 102. In such an embodiment, controller 44 need not be configured to perform this check. In embodiments where manager 24 is provided, database 42 may be omitted as part of one or more of it is equipment units 22. In some embodiments, manager 24 may itself include control instructions 40, wherein controller 104, following the control instructions contained in manager 24, directly controls force receiving members 30. In such an embodiment, one or more of fitness equipment units 22 may omit control instructions 40. As a result, fitness equipment units 22 may be simplified.

In operation, manager 24 enables a person to view his or her entire subscription plan for multiple fitness equipment units 22. Manager 24 further enables a person to revise or upgrade his or her plan for one or more of the fitness community 22 being managed by manager 24. In other embodiments, manager 24 may be omitted.

Figure 3 illustrates one example display presentation 110 that may be presented by controller 104 on display 92 based upon the identification of the person exercising and his or her current subscription plan as retrieved from subscription database 102. In other embodiments, controller 44 may retrieve a subscription plan 106 from database 102 and provide presentation 110 on display 32 at a particular fitness equipment unit 22. As shown by Figure 3, presentation 110 identifies the current subscription plan 112 for multiple fitness equipment units 22A or 22B. In the example illustrated, the current subscription plan 112 includes current accessibility (A), available parameters (AP) and available video or visual presentations (AVP) (each of which is discussed above) for each of the fitness equipment units FEU1 to FEUN. The presentation of the current subscription plan 112 for the person exercising allows the person to see his or her current options. In embodiments where subscription plan 102 is presented at the particular fitness equipment unit 22 on display 32, the user person may select one or more of the options using selector input 36.

According to one embodiment, controller 104 may further generate control signals causing display 92 (or display 32) to present various upgrade options or offers 114. As shown by Figure 3, the upgrade offers may include offers to upgrade accessibility A to one or more of fitness equipment units 22A, 22B such as increasing either one or more quantity limitations Q or universal time limitations T. The upgrade offers may include one or more additional parameters (P) or one or more additional visual presentations (VP). In addition, the upgrade offers may include prepackaged sets (Package) of upgrade options, wherein each Package includes a different group of accessibility, additional available parameters and additional available visual presentations. With each offer, presentation 110 displays a price ($)for the upgrade. The cost or price of such upgrades may constitute a one-time payment, a monthly payment, yearly payment of the like.

If the user wishes to upgrade his or her subscription plan for one or more fitness equipment units 22, the person may select the offer using selector input 96 and may remit payment using payment input 98. In one embodiment, actual payment may not be required, wherein the person merely authorizes his or her account to be billed or charged. In some embodiments, the person at one fitness equipment unit 22 may upgrade or revise his or her subscription plan to include additional features for use on a different fitness equipment unit 22. Once an upgrade is selected and paid or charged, controller 104 updates the person subscription plan 106 in subscription database 102. Databases 42 in the individual fitness equipment units 22 may also be updated by either controller 104 or controller 44.

At predetermined times, controller 104 further maintains subscription database 102 by comparing the current date or time with any expiration date for any feature in the subscription plan. Controller 104 culls or removes features from the subscription plan that have expired. In some embodiments, controller 104 may additionally modify any subscription plans contained in database 42 at each of the one or more fitness equipment units 22 to reflect the expiration of such features. In embodiments where subscriptions have a termination or expiration date, controller 104 may additionally generate control signals causing visual presentation 110 (and/or visual presentation 60 at an individual fitness equipment unit 22) to additionally inform the user of features currently in the current subscription that are about to expire.

Subscription monitor 26 comprises an electronic device configured to monitor an individual person's subscription plan as stored in either a database 42 of an individual fitness equipment unit 22 or database 102 of manager 24. Subscription monitor 26 facilitates viewing, upgrading or maintaining of a person's subscription plan for an individual fitness equipment unit 22 or other multiple fitness equipment units 22. Fitness equipment unit 22 communicates with each of manager 24 and one of more fitness equipment units 22 in a wired or wireless fashion (such as through the Internet or intranet). In one embodiment, monitor 26 may comprise a portable electronic device such as a cell phone, tablet, personal data assistant (PDA), laptop and the like. In another embodiment, 26 may comprise a desktop computer, computing television and the like.

As shown by Figure 1, monitor 26 comprises display 132, selector input 136, payment input 138 and controller 144. Display 132, selector input 136 and payment input 138 are substantially similar to display 92, selector input 96 and unit input 98 described above. Controller 144 comprises one or more processing units configured to follow instructions contained in a memory structure. Controller 414 and the associated instructions are configured to retrieve a subscription plan 54 from database 42 or to retrieve the subscription plan 106 from database 102 of manager 24, wherein the retrieved subscription plan is displayed on display 132. In one embodiment, controller 144 is further configured to allow a person to upgrade, modify maintain his or her restriction plan using selector input one 136 and payment input 138. In some embodiments, controller 144 may additionally be configured to store a retrieved or modify subscription plan in a memory structure within monitor 26. In some embodiments, monitor 26 may be omitted.

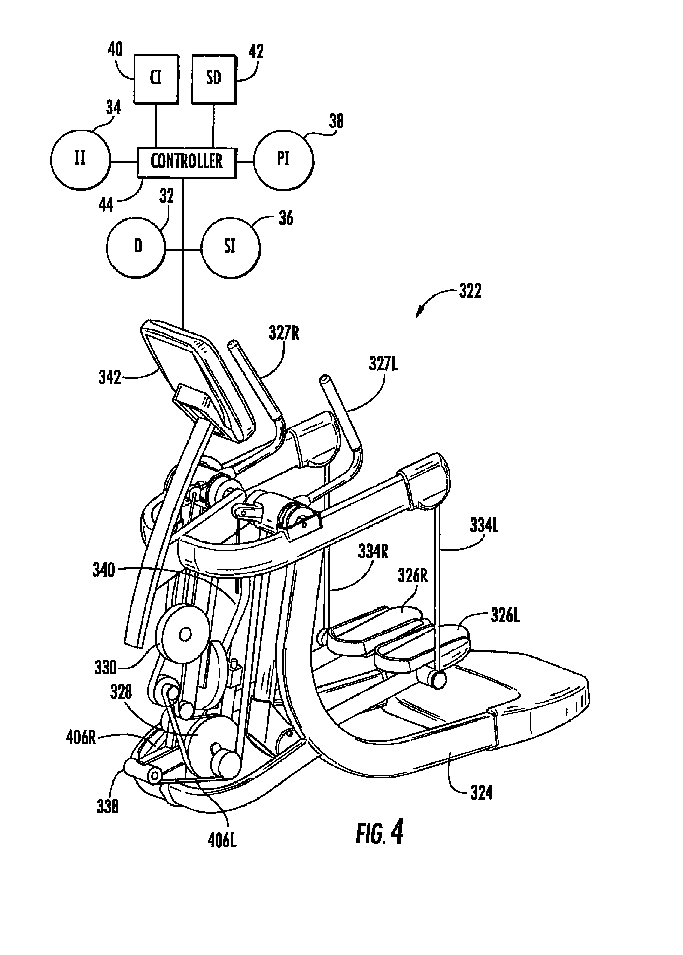

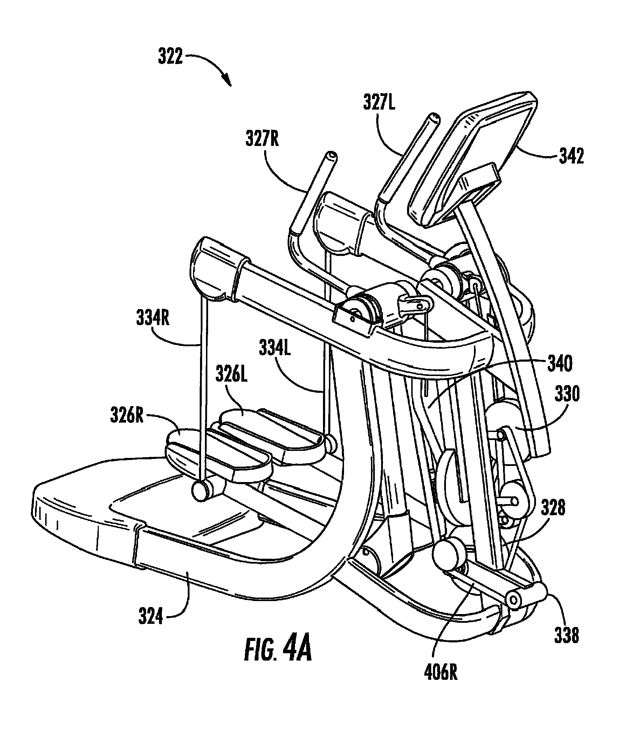

Figures 4-10 illustrate one example embodiment of the particular fitness equipment unit 910 which comprises an adaptive motion machine. In alternative preferred embodiments, other types of exercise machines, including both cardiovascular exercise machines/equipment and weight lifting/strength machines/equipment, providing variable two and/or three dimensional paths of motion for the upper and/or lower body of the user can also be used. Exercise device or apparatus 320 allows a person to adjust a horizontal length of his or her stride simply by the person applying force to foot supports of the fitness equipment unit. Fitness equipment unit 322 further allows the person to also adjust a vertical length or vertical step height. Fitness equipment unit 322 provides such freedom of motion using flexible elements 404 and 406 in an architecture that is compact, less complex and less expensive.

As shown by Figures 4-10, fitness equipment unit 322 comprises frame 324, linkage assemblies 326L, 326R (collectively referred to as linkage assemblies 326), swing arms 327R, 327L (collectively referred to as swing arms 327), crank system 328, resistance system 330, coupling systems 334L, 334R (collectively referred to as coupling systems 334), step height adjustment mechanism 338, horizontal resistance system 340 and interface panel 342.

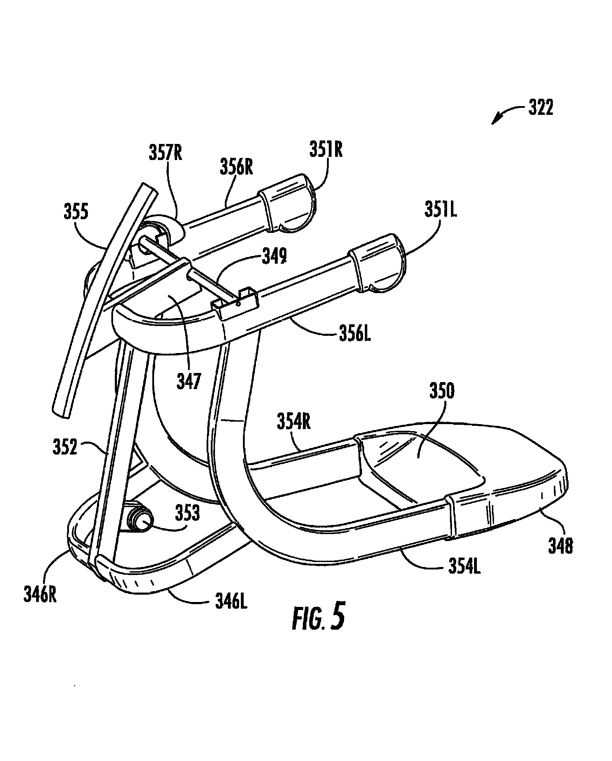

Frame 324 supports fitness equipment unit 322 upon a base or floor. As illustrated in Figures 4A and 5, frame 324 includes rear base portion 350, front or forward post or leg 352, rear supports or legs 354R, 354L (collectively referred to as rear supports 354), side arms 356L, 356R (collectively referred to as side arms 356), front support 355, front supports 346R, 346L (collectively referred to as front supports 346), front support 347, cross-shaft 349, end caps 351 R, 351 L (collectively referred to as end caps 351), covers 357R, 357L (collectively referred to as covers 357) and crank support 353. Base portion 350 bears against the floor and is connected to rear supports 354. The bottom of forward post 352 bears against the floor. Forward post 352 extends at a forward end of fitness equipment unit 322 and is connected to and supports front support 347. Front support 347 connects to and supports side arms 356 and cross-shaft 349. Front supports 346 connect front post 352 to rear supports 354. Platform 348 connects to rear supports or legs 354 and covers rear support 350. Front support 355 connects to front support 347 and supports interface panel 342. Side arms 356 and front support 347 support cross-shaft 349. Rear supports or legs 354 extend toward the rear end of fitness equipment unit 322 and are connected to side arms 356. End caps 351 R, 351 L (collectively referred to as end caps 351) and covers 361R, 361L (collectively referred to as covers 361) connect to side arms 356.

Side arms 356 extend rearwardly from leg 352 and front support 347 on opposite sides of both linkage assemblies 326. Side arms 356 extend substantially parallel to one another at the same vertical height. Side arms 356 provide bars, beams or shafts by which a person's left and right hands may grasp or rest upon when mounting fitness equipment unit 322 or when otherwise not grasping handle portions 366R, 366L (collectively referred to as handle portions) of swing arms 327. Side arms 356 help retain a person on linkage assemblies 326 and on fitness equipment unit 322 and reduce the likelihood of a person falling off of fitness equipment unit 322. Side arms 356 assist in supporting cross-shaft 349 and portions of coupling systems 334. Side arms 356 further serve as shields about flexible elements of couplings systems 334. End caps 351 and covers 357 cover portions of coupling systems 334 by attachment to side arms 356.

Forward post 352 supports front support 347, crank support 353, resistance system 330, step height adjustment mechanism 338 and horizontal resistance system 340. For ease of illustration, portions of post 352, such as brackets or support plates extending forwardly from post 352 are omitted.

Cross-shaft 349 supports linkage assemblies 326, swing arms 327 and portions of coupling assemblies 334. Front supports 346 provide additional support between front post 352 and rear supports 354.Crank support 353 supports portions of crank system 328 and portions of step height adjustment mechanism 338. Crank support 353 comprises a plate, beam, bar, channel or similar element firmly attached to the rearward side of front post 352. Crank support 353 also comprises operable attachment elements for portions of crank system 328 and step height adjustment mechanism 338. Such operable attachment elements include shafts, hubs, collars, pins, levers or similar elements to allow for movement of crank system 328 potions and step height mechanism 338 portions around a horizontal centerline 374. In another embodiment, support for portions of step height mechanism 338 may be omitted from crank support 353. In some embodiments, crank support 353 may be attached forward of front post 352 or be supported by other portions of frame 324.

Platform 348 provides a location from which the user of fitness equipment unit 322 may mount foot pads 362R, 362L (commonly referred to as foot pads) of linkage assemblies 326.

Linkage assemblies 326 comprise one or more members movably supported by frame 324 and configured to elevate and support a person's feet as the person exercising applies force to such linkage assemblies to move such linkage assemblies relative to frame 324. Linkage assemblies 326 are coupled to one another so as to automatically move 180 degrees out of phase with respect to one another when opposing forces are applied to linkage assemblies 326. The person exercising exerts force on foot pads 362 and foot support members 360, alternating right and left, while also pushing and pulling on linkage assemblies 326 to create the out of phase movement of linkage assemblies 326. In other embodiments, other means of synchronization may be used.

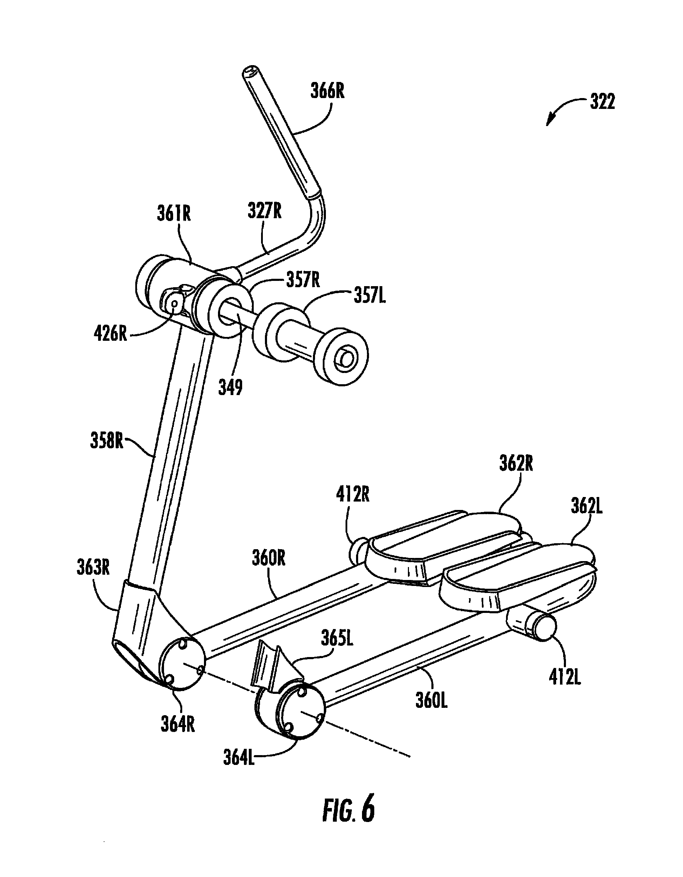

As illustrated in Figure 6, each of linkage assemblies 326 includes motion members 358R, 358L (collectively referred to motion members 358), torque bars 359R, 359L (collectively referred to torque bars 359), foot support members 360R, 360L (collectively referred to as foot support members 360), hubs 361 R, 361 L (collectively referred to as hubs 361), foot pads 362R, 362L (collectively referred to as foot pads 362), saddles 363R, 363L (collectively referred to as saddles 363), joints 364R, 364L (collectively referred to as joints 364) and joint covers 365R, 365L (collectively referred to as joint covers 365).

Torque bars 359 are supported by cross-shaft 349. Torque bars 359 are spool-shaped including a center portion of one diameter and end portions of diameters larger than the diameter of the center portion. Each of torque bars 359 includes a circular hole located on its radial centerline and extending along its entire length. The inside diameter of the circular hole is slightly larger than the outside diameter of cross-shaft 349. Torque bars 359 mount on to cross-shaft 349 such as to allow rotational movement of torque bars 359 on cross-shaft 349. The rotational movement of torque bars 359 creates resulting rotational movement or winding and unwinding of portions of coupling systems 334.

Each of hubs 361 is a circular element with a hollow center that is mounted on the smaller diameter portion of one of torque bars 359. Hubs 361 pivotally connect swing arms 327 and motion members 358. The rearward sides of hubs 361 are attached to swing arms 327. The bottom sides of hubs 361 are attached to motion members 358. The forward sides of hubs 361 are attached to portions of coupling systems 334.

Motion members 358 are essentially vertical components that transfer movement from hubs 361 to lower portions of linkage assemblies 326. Motion members 358 are attached to saddles 363 and joint covers 365. Each of saddles 363 wrap around the forward side of the lowest part of one of motion members 358 and are attached to motion members 358. Each of saddles 363 has one or more arms that attach to joints 364. Each of joint covers 365 attach to the rearward side of one of motion members 358 immediately above joint 364. The combination of saddles 363, joints 364 and joint covers 365 pivotally connect motion members 358 to foot support members 360. In other embodiments, motion members 358 and foot support members 360 may be pivotally connected other means such as knee braces, welded hubs or the like.

Each foot support member 360 (also known as a stair arm) serves as a force receiving member and extends substantially horizontally from one of joints 364 and supports one of foot pads 362. Each foot pad 362 comprises a paddle, pedal, or the like providing a surface upon which a person's foot may rest. Each foot pad 362 further includes a toe cover or toe clip against which a person's foot or toes may apply force in an upward or vertical direction. Foot pads 362 may have a variety of different sizes, shapes and configurations. In other embodiments, each motion member 358 and foot support member 360 (sometimes referred to as a foot link) may also have different configurations, shapes and connections. For example, in other embodiments, in lieu of foot support member 360 having a rear end which is cantilevered, foot support member 360 may alternatively have a rear end which is pivotally supported by another supporting linkage extending from one of side arms 356 or another portion of frame 324.

Swing arms 327 comprise arms having handle portions 366 configured to be grasped by a person while linkage assemblies 326 are pivoted relative to frame 324. In the example illustrated, swing arms 327 are rigidly connected to hubs 361 which are also rigidly connected to motion members 358. Swing arms 327, hubs 361 and motion members 358 comprise a fixed arrangement that pivots around cross-shaft 349. As a result, swing arms 327 permit a person to exercise his or her arms and upper body. In other embodiments, swing arms 327 may pivot independent of linkage assemblies 326, may have independent resistance systems for exercising the upper body or may be rigidly or stationarily supported by frame 324. In some embodiments, swing arms 327 may be omitted.

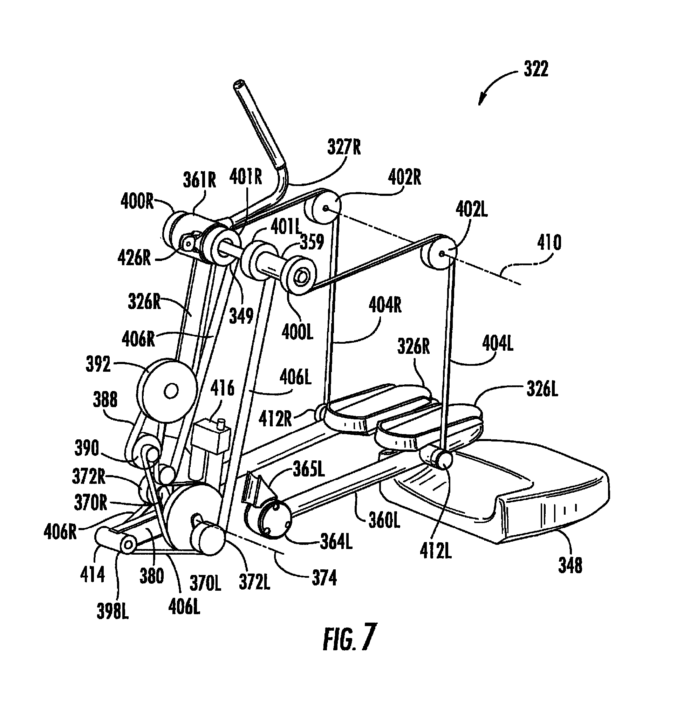

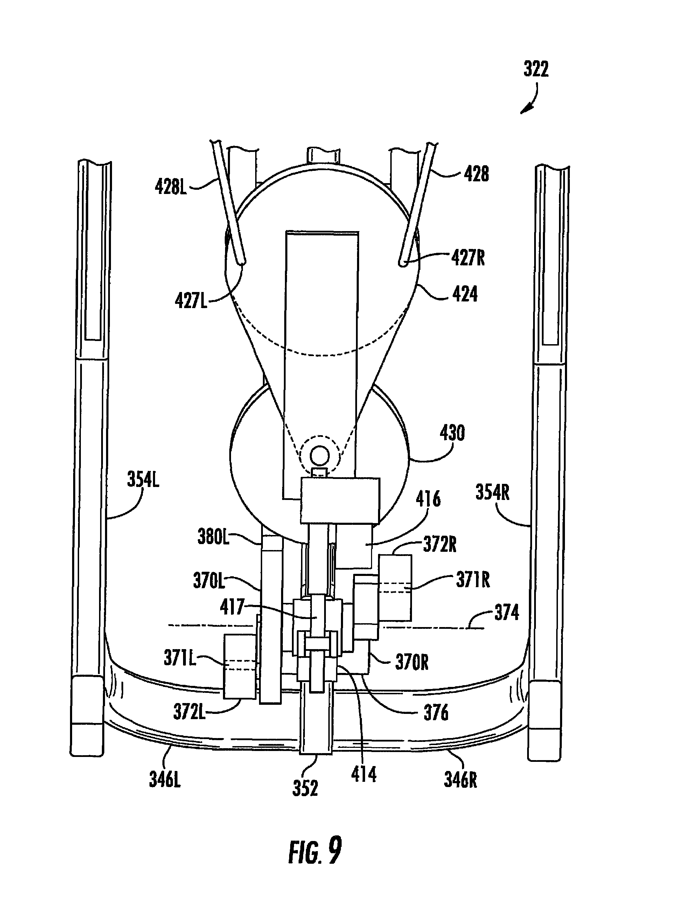

Figures 7 and 9 illustrate crank system 328 in more detail. Flexible element portions of coupling systems 334 are omitted from Figure 9 for ease of illustration. Crank system 328 comprises a mechanism configured to synchronize movement of linkage assemblies 326 and to apply a resistance to such movement. As shown by such figures, crank system 328 crank arms or cranks 370R, 370L (collectively referred to as crank arms 370), crank guide arms 371 R, 371 L (collectively referred to as crank guide arms 371), flexible element crank guides 372R, 372L (collectively referred to as flexible element crank guides 372) and crank shaft 376.

Cranks 370 transfer force and movement from coupling systems 334 to resistance system 330. Cranks 370 are attached to and supported by crank shaft 376. Crank shaft 376 is supported by crank support 353 in a manner to allow rotation of crankshaft 376 and cranks 370 about horizontal axis 374. Because cranks 370 rotate about a substantially horizontal axis 374 which is positioned near forward post 352, crank system 328 is more compact. In yet other embodiments, crank system 328 may be located elsewhere within the confines of frame 324.

In the example illustrated, crank 370L comprises a combined input crank and sheave in the form of a disk, wheel or the like, wherein the disc or wheel concentrically extends about axis 374. In other embodiments, crank 370L may comprise one or more members configured to rotate about axis 374, wherein crank 370L does not concentrically extend about axis 374. In other embodiments, crank 270L may rotate about a vertical axis in a manner such as illustrated for fitness equipment unit 20.

Crank 370R is fixed to crank 370L so as to rotate with crank 370L. In the example illustrated, crank 370R comprises an arm radially extending outward from shaft 376 and supporting guide 372R towards its outer radial end. Crank 370R supports flexible element crank guide 372R attached to crank arm 370R at crank guide arm 371 R. Crank 370L includes flexible element crank guide 372L attached to crank arm 370L at crank guide arm 371 L.

Crank guide arms 371 and flexible element crank guides 372 are located on crank arms 370 at points that are equidistant and radially spaced from axis 374. The locations of crank guide 372R and crank guide 372L are positioned 180 degrees out of phase from each other. Flexible element crank guides 372 comprise members that are connected to and carried by cranks arms 370 so as to rotate about axis 374 and about which front flexible elements 406 (406R, 406L) of coupling system 334 wrap so as to transmit force to crank guides 372 and ultimately to cranks 370. In the example illustrated, flexible element crank guides 372 comprise a pulley. In other embodiments, flexible element crank guides 372 may alternatively comprise a spool or disc against which a flexible element moves or slides without rotation of the flexible element crank guide 372.

Resistance system 330 applies additional resistance to the rotation of crank system 328. In the particular example illustrated, resistance system 330 provides a selectively adjustable incremental resistance to the rotation of cranks 369 of crank system 328. Resistance system 330 includes belt 380, speed changer 390, belt 388 and resistance source 392. In the illustrated embodiment, speed changer 390 comprises a step up pulley. Belt 380 wraps about one of cranks 369 and the smaller wheel of speed changer 390. Belt 388 wraps about the larger wheel of speed changer 390 and also about the shaft of resistance source 392. The attachment of resistance source 392 to front post 352 adjacent to cranks 369 and with horizontal axis of rotation allows for a more compact and efficient design for fitness equipment unit 322. In other embodiments, chain and sprocket arrangements, gear trains and other transmissions may be used to operatively couple cranks 370 to resistance source 392.

Resistance source 392 comprises a mechanism configured to rotate against a selectively adjustable resistance. In one embodiment, resistance source 392 comprises a metal plate and one or more magnets forming an Eddy brake. In one embodiment, the one or more magnets comprise electromagnets, allowing the strength of the magnetic force to be selectively adjusted to control and vary the resistance applied against the rotation of cranks 370. In another embodiment, resistance source 392 may comprise an electric generator. In still another embodiment, resistance source 392 may comprise two surfaces in frictional contact with one another to apply a frictional resistance against rotation of cranks 370. In another embodiment, air brakes may be utilized. In still other embodiments, other brakes or resistance mechanisms may be utilized.

Because resistance system 330 utilizes a two-stage transmission between cranks 369 and resistance source 392, the arrangement or architecture of crank system 328 and resistance system 330 is more compact and the speed ratio between cranks 369 and resistance source 392 (approximately 12:1) provides improved electric performance. In other embodiments, a single stage or a transmission with greater than two stages may be employed. In yet other embodiments, resistance system 330 may have other configurations or may be omitted. For example, in another embodiment, the transmission of resistance system 330 may include gear trains, chains and sprockets or the like.

As best shown by Figures 4, 4A and 7, coupling system 334 operably couples or joins step height adjustment system 338 to foot support members 360 or footpads 362. Coupling systems 334 include front end flexible element mounts 398R, 398L (collectively referred to as front end flexible element mounts 398), front flexible elements 406R, 406L (collectively referred to as front flexible elements 406), torque bar inboard flexible element mounts 401 R, 401 L (collectively referred to as torque bar inboard flexible element mounts 401), torque bar outboard flexible element mounts 400R, 400L (collectively referred to as torque bar rear flexible element mounts 404), rear flexible elements 404R, 400L (collectively referred to as rear flexible elements 404), rear guide elements 402R, 402L (collectively referred to as rear guide elements 402 and foot pad flexible element mounts 412R, 412L (collectively referred to as foot pad flexible element mounts 412).

Front flexible elements 406 and rear flexible elements 404 comprise flat belts of fiber reinforced polymer. In one embodiment, elements 404 and 406 comprise Kevlar reinforced polyurethane. Fiber reinforced polymer provides the advantage of durability for flexible elements 404 and 406. In another embodiment, one or more of front flexible elements 406 and rear flexible elements 404 may comprise bendable members such as cables, wires, ropes, belts, cords, strings, chains, and the like. In another embodiment, one or more of front flexible elements 406 and rear flexible elements 404 may comprise belts of materials other than fiber reinforced polymer.

As shown by Figure 7, front end flexible element mount 398 (also known as a "dead end") comprises a mount or securement point at which an end of front flexible element 406 is attached. In the example illustrated, end mount 398 for each of coupling systems 334 is provided by step height adjustment mechanism 338. In other embodiments in which step height adjustment mechanism 338 is omitted, front end flexible element mount 398 may be provided by part of frame 324. In still other embodiments in which the ends of flexible elements 406 are directly attached to cranks 369 and do not wrap about a flexible elements crank guide 372, end mounts 398 may be provided on cranks 369.

Torque bar inboard flexible element mounts 401 comprise the spool ends of torque bars 359 that are located nearest to the longitudinal centerline of cross-shaft 349. Torque bar outboard flexible element mounts 400 comprise the spool ends of torque bars 359 that are located nearest to the longitudinal ends of cross-shaft 349.

Front flexible elements 406 wrap around flexible elements crank guides 372 and also wrap around from below and toward the rearward side of torque bar inboard flexible element mounts 401. As viewed from the left side of fitness equipment unit 322, front end flexible elements 406 wrap around torque bar inboard flexible elements mounts 401 in a counter-clockwise direction. The rearward ends of front flexible elements 406 attach to torque bar inboard flexible element mounts 401. The forward ends of rear flexible elements 404 attach to torque bar outboard flexible elements mounts 400. Rear flexible elements 404 wrap from above and toward the forward side of torque bar outboard flexible element mounts 400 in a counter-clockwise direction as viewed from the left side of fitness equipment unit 322. The method of attachment of front flexible elements 406 to torque bar inboard flexible elements mounts 401 and of rear flexible elements 404 to torque bar outboard flexible element mounts 400 serves to laterally transmit torque back and forth between elements 406 and 404 through torque bar 359 in an wind/unwind motion.

A shown by Figure 7, the torque bar flexible element mounts 400 guide and direct movement of the rear flexible elements 404 to the interior of side arms 356 and toward rear guide elements 402.

In the example illustrated, rear guide elements 402 comprise pulleys rotationally supported by side arms 356 of frame 324 proximate to a rear end of fitness equipment unit 322 substantially vertically above footpads 362 when footpads 362 are longitudinally aligned. In other embodiments, each of rear guide elements 402 may alternatively comprise a low friction surface which does not rotate and against which flexible elements 404 moves or slides.

As shown by Figure 7, each of guide elements 402 further guides and directs flexible element 404 through an opening from an interior of side arm 356 in a substantially vertical direction down to foot support members 360 and footpads 362. In the example illustrated, guide elements 402 rotate about a substantially horizontal axis 410. Although coupling systems 334 are illustrated as having one guide element 402, in other embodiments, coupling systems 334 may alternatively include a greater or fewer of such guide elements.

In the example illustrated, the rearward end of rear flexible elements 404 is fixed to a foot support member 360 by a mount 412 at a location transversely opposite to footpad 362 near or proximate to a forward end of footpad 362. In the example illustrated, each mount 412 includes a body that slides (via screw adjustment) up and down relative to a pivoting block attached to the associated member 360, wherein flexible element 404 is fixed or secured to the body of the mount. Each mount 412 allows the location of members 360 to be adjusted so as to be level with one another. In other embodiments, mounts 412 may comprise other securement mechanisms such as clamps, fasteners and the like. In another embodiment, flexible element 404 may be clamped to mount 412 as described herein for fitness equipment unit 20.

Each rear flexible element 404 extends from mount 412 in a substantially vertical direction until engaging rear guide 402. Rear flexible element 404 wraps partially about rear guide element 402 into an interior of one of side arm 356. Rear flexible element 404 extends through the interior of side arm 356 until engaging torque bar outboard flexible element mount 400. Movement is translated from the rear flexible element 404 to the front flexible element 406 through torque bar 359. Front flexible element 406 extends from torque inboard flexible element mount 401 and wraps around flexible elements crank guides 372. Finally, the front end of each front flexible element 406 is secured to one of front end mounts 398.

Because each of coupling systems 334 employs flexible elements (404 and 406) rather than rigid inflexible members or elements, forces may be more smoothly transmitted across convoluted paths, allowing coupling systems 334 and crank system 328 to be more compactly arranged and to be less complex and expensive. In addition, flexible elements (404 and 406) also have a reduced diameter as compared to rigid elements which permits the transmission of forces from linkage assemblies 326 to crank system 328 in even a more compact fashion. In other embodiments, at least segments or portions of front flexible elements 406 or rear flexible elements 404 may alternatively be replaced with rigid inflexible members or elements.

Step height adjustment mechanism 338 is configured to provide foot support members 360 and foot pads 362 with a multitude of different user selectable maximum upper and lower vertical ranges of motion. Adjustment mechanism 338 allows a person to adjust a maximum step height or a maximum step depth of a path through which the left and right foot supports 360 may move.

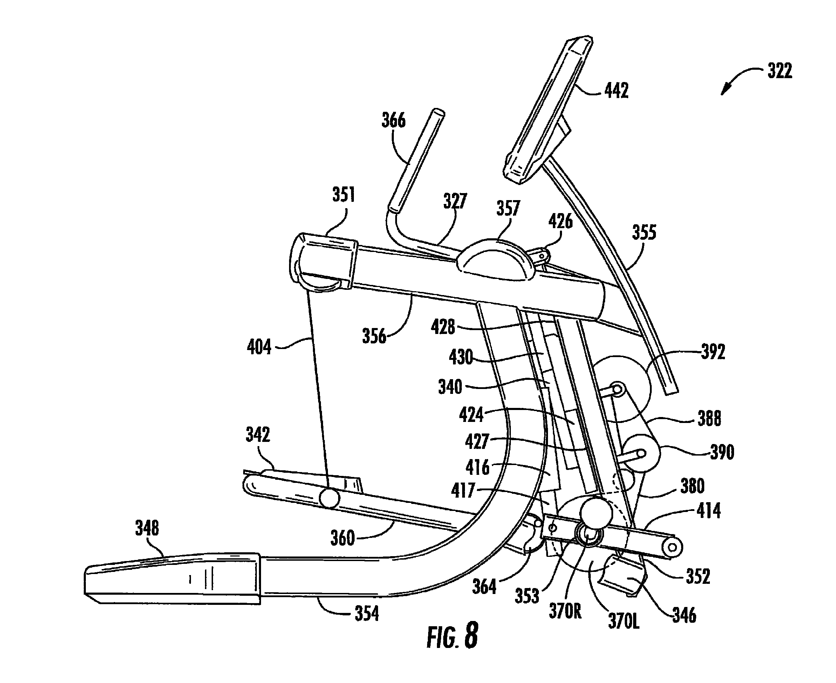

As shown by Figures 8-10, step height adjustment mechanism 338 comprises adjustment member 414 and actuator 416 connected by linkage 417. Step height adjustment mechanism 338 changes the location of front end flexible element mounts 398 which, in turn, modifies the paths of front flexible elements 406 and rear flexible elements 404 and adjusts the positions of foot pads 362.

Adjustment member 414 pivots vertically about a horizontal axis at the center of its attachment to frame 324. Front end flexible elements mounts 398 are located on the forward end of adjustment member 414. The rearward end of adjustment member 414 is connected to actuator 416 by linkage 417. As viewed from the left side of fitness equipment unit 322, movement of linkage 417 downward pivots adjustment member 414 in a clockwise direction which increases the vertical position of front flexible element mounts 398. In the illustrated example, the pivot axis of adjustment member 414 is coincident with axis 374 of crank system 328. As a result, movement of front end flexible end mounts 398 from the lowest position to the highest position results in an increase in the overall step height or distance with a majority of the increase occurring at the upper end of the range of motion. In other words, the upper end or highest vertical height attained by the footpads 326 during their motion will rise by an extent nearly equaling the total increase in step height distance. The lowest point to which the footpads 326 fall in only minimally lowered. By way of example, it the step height or range is increased by a distance X, the highest vertical point of foot pads 326 may increase by a distance 4/5 X which the lowest vertical height will only fall by a distance 1/5 X. As a result, linkage assemblies 320 may be supported at a lower elevation with a reduced risk of the linkage assemblies 320 or their footpads 326 bottoming out as a result of step height adjustment.

In other embodiments, adjustment member 414 and crank system 328 may pivot or rotate about different axes. For example, the axis of adjustment member 414 and crank system 328 may be offset such that changes in the step height or step range (the distance between the highest and lowest points in the path of foot pads 326) are equally distributed such that an increase or decrease in step height or range will result in the highest vertical point and the lowest vertical point of the path of pads 326 being raised and lowered by substantially equal amounts. In yet other embodiments, the axis of adjustment member 414 and crank system 328 may be offset such that changes in the step height or step range are largely achieved at the lower end of the range of motion, the lowermost elevation changing by a much larger extent as compared to the extent to which the uppermost elevation of foot pads 326 changes.

Although front end flexible element mounts 398 are illustrated as moving in unison, front end flexible element mounts 398 may be supported so as to be movable independent of one another to different locations - either by being rotated or by being translated. In yet other embodiments, step height adjustment member may move linearly through a slotted or sliding mechanism or the like. Overall, the location of step height adjustment mechanism 338 on front post 352 with vertical movement of front end flexible element mounts 398 provides a more compact and efficient design.

Actuator 416 and linkage 417 comprise a mechanism configured to rotate or move the adjustment member 414 between a plurality of different positions so as to position and retain front end flexible element mounts 398 at different positions with respect to frame 324, cranks 369 and flexible element crank guides 372. In one embodiment, actuator 416 comprises a motor configured to rotationally drive a threaded shaft or screw threadably engaging a nut or internally threaded member connected to member 414. Rotation of the threaded shaft or screw results in member 414 being raised and lowered and pivoting about axis 374. In other embodiments, actuator 416 and linkage 417 may comprise other means for raising and lowering member 414. For example, actuator 416 may alternatively comprise a hydraulic or pneumatic piston and cylinder assembly. In yet another embodiment, after 416 may comprise an electric solenoid. In still other embodiments, actuator 416 may comprise various gears or cam arrangements.

Although actuator 417 is illustrated as being attached to frame 324 rearward of post-352 and being further attached to member 414 rearwardly of the pivot axis of member 414, in other embodiments, actuator 417 may alternatively be attached to the member 414 forwardly of the pivot axis of member 414, on the same side of the pivot axis as mounts 398. In yet other embodiment, actuator 417 may be supported on the forward side of front post 352 or on another part of frame 324.

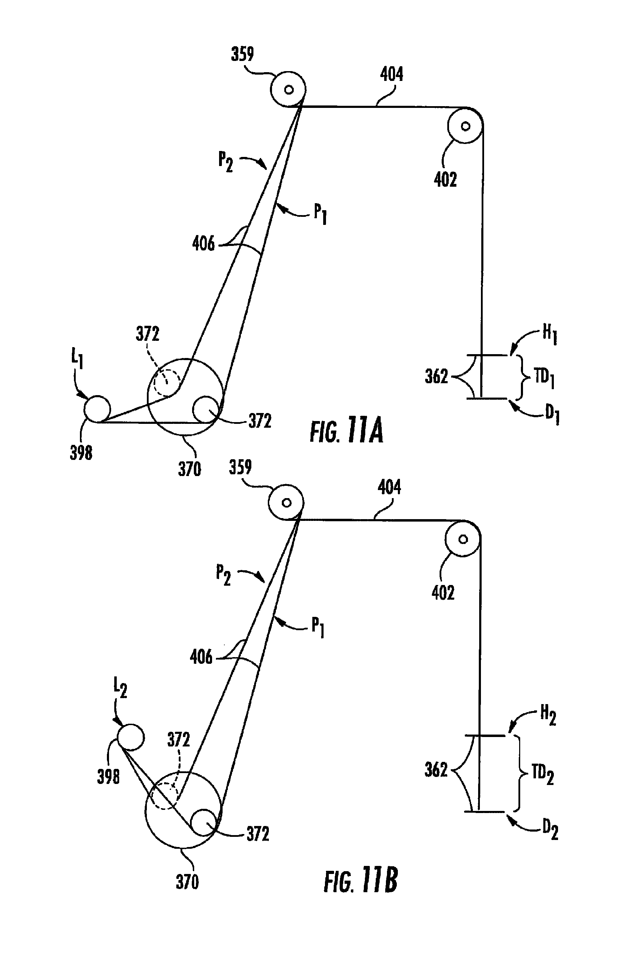

Figures 11A and 11B diagrammatically illustrate the adjustment of travel distance achieved by the repositioning of front end flexible elements mounts 398. Both figures present an approximate elevation view of select components of step height adjustment mechanism 338, crank system 328, coupling system 334 and linkage assemblies 326. As shown by Figures 11A and 11B, repositioning front end flexible element mount 398 varies the amount or extent by which the front flexible element 406 wraps about the associated flexible element crank guide 372. This change in the amount of wrap changes the travel distance or travel range of foot supports 362. In one embodiment, the maximum step height, maximum step depth or both maximum step height and depth of the path through which footpads 362 may be adjusted.

Figure 11A illustrates the approximate orientation of components when adjustment member 414 is pivoted to position front end flexible elements mounts 398 at their lowest point, L1. The resulting step height is "Low Travel Distance", TD1, which is the difference in the location of one of foot pads 362 at point H1 and the location of the other foot pad 362 at point D1. Figure 11 B illustrates the approximate orientation of components when adjustment member 414 is pivoted to position front end flexible elements mounts 398 at their highest point, L2. The resulting step height is "High Travel Distance", TD2, which is the difference in the location of one of foot pads 362 at point H2 and the location of the other foot pad 362 at point D2.

As illustrated by Figure 11A, when front end flexible element mount 398 is at the lowest position L1, the combination of front flexible element 406 and rear flexible element 404 on one side of fitness equipment unit 322 extends along path P1 resulting in foot pad 362 location at position H1. The combination of front flexible element 406 and rear flexible element 407 on the opposing side of fitness equipment unit 322 extends along path P2 resulting in foot pad 362 at position D1. The distance between the first foot pad 362 position H1 and the second foot pad 362 position D1 is TD1, "Low Travel Distance". TD1 represents the minimum step height.

As illustrated by Figure 11 B, when front end flexible element mount 398 is at the highest position L2, the combination of front flexible element 406 and rear flexible element 404 on one side of fitness equipment unit 322 extends through path P3 resulting in foot pad 362 position at H2. The combination of front flexible element 406 and rear flexible element 404 on the opposing side of fitness equipment unit 322 extends along path P4 resulting in foot pad 362 position D2. The distance between the first foot pad 362 position H2 and the second foot pad 362 position D2 is TD2, "High Travel Distance". TD2 represents the maximum step height.

During pivoting of adjustment member 414, the amount of wrap of front flexible elements 406 around flexible element crank guides 372 changes. As the vertical location of front end flexible element mounts 398 rises from L1 toward L2, the amount of wrap increases which, in turn, changes the path of front flexible elements 406.

Each front flexible element 406 interfaces with a corresponding rear flexible element 404 at a torque bar 359. Front flexible element 406R wraps around and attaches to the torque bar inboard flexible element mount 401 R. Rear flexible element 404R wraps around and attaches to torque bar outboard flexible element mount 400R. Rotation of the torque bars 359 around cross-shaft 349 translate movement between front flexible element 406 and rear flexible element 404. The total path length of each combination of front flexible element 406 and rear flexible element 404 remains essentially unchanged. A change in the position of the front flexible element mount 398 will result in a corresponding change to the position of foot pad flexible element mount 412, which repositions foot pads 362.

Increasing the wrap angle of front flexible element 406 around flexible element crank guide 372 increases the mechanical advantage of the user on the crank. Conversely, decreasing the wrap angle reduces the mechanical advantage of the user on the crank. By adjusting the position of front end flexible element mount 398, the maximum height and/or the maximum depth to which foot pad 362 may be raised or lowered may be adjusted. Likewise, the total range or total travel distance through which foot pad 362 is moved may also be adjusted

Adjustment member 414 can be pivoted to a continuum of different positions and may be retained in any one position along the continuum. In other embodiments, adjustment member 414 may alternatively rotate between a multitude of distinct discrete spaced positions at various predetermined angles about its pivot point. In such an alternative embodiment, notches, detents or other retention mechanism may be used to define the distinct spaced positions at which adjustment member 414 may be retained.

Actuator 416 comprises a mechanism configured to move adjustment member 414. In the example illustrated, actuator 416 comprises a powered actuator driven by electrical power. In one embodiment, actuator 416 comprises an electric powered motor configured to drive a worm or lead screw arrangement to generate linear translation so as to rotate adjustment member 414 about axis 374. In yet another embodiment, actuator 416 may comprise an electric motor, such as a stepper motor, servomotor and the like, directly connected to a shaft secured to adjustment member 414 along axis 374 or connected to a shaft secured to adjustment member 414 by speed reducing device or gear train to selectively rotate adjustment member 414. In still other embodiments, actuator 416 may comprise electric solenoid or a hydraulic or a pneumatic piston-cylinder assembly operably coupled to adjustment member 414 so as to rotate adjustment member 414.

According to one embodiment, powered actuator 416 repositions adjustment member 414 to adjust the step height in response to control signals from a controller 446 associated with interface panel 442. In one embodiment, such adjustment may be in response to a person depressing a button, sliding a slider bar, actuating a switch, entering a voice command to voice recognition software through microphone or other input. In another embodiment, such adjustment may be in accordance with a preprogrammed or predetermined exercise routine stored in memory, wherein the step height is to be adjusted during an exercise routine. Because such adjustment is powered and does not require a person to detach or disassemble any portion of fitness equipment unit 322, such adjustment may be made "on-the-fly" during exercise as foot pads 362 are moving along a path. In other words, an exercise routine or workout need not be interrupted.

In other embodiments, actuator 416 may alternatively comprise a non-powered actuator. For example, actually 416 may alternatively be configured to be manually powered, wherein force or motion applied by a person is mechanically transmitted to adjustment member 414 to reposition adjustment member 414. After adjustment, adjustment member 414 may be retained in place by one or more hooks, clamps, catches, detents or friction surfaces.

Although adjustment member 414 is illustrated as being rotated so as to reposition end mounts 398 and so as to adjust the step height of fitness equipment unit 322, in other embodiments, the positioning of end mounts 398 may be adjusted in other fashions. For example, in another embodiment, end mounts 398 may alternatively be linearly movable or configured to slide or translate between different positions relative to frame 324 and relative to crank flexible element guides 372.

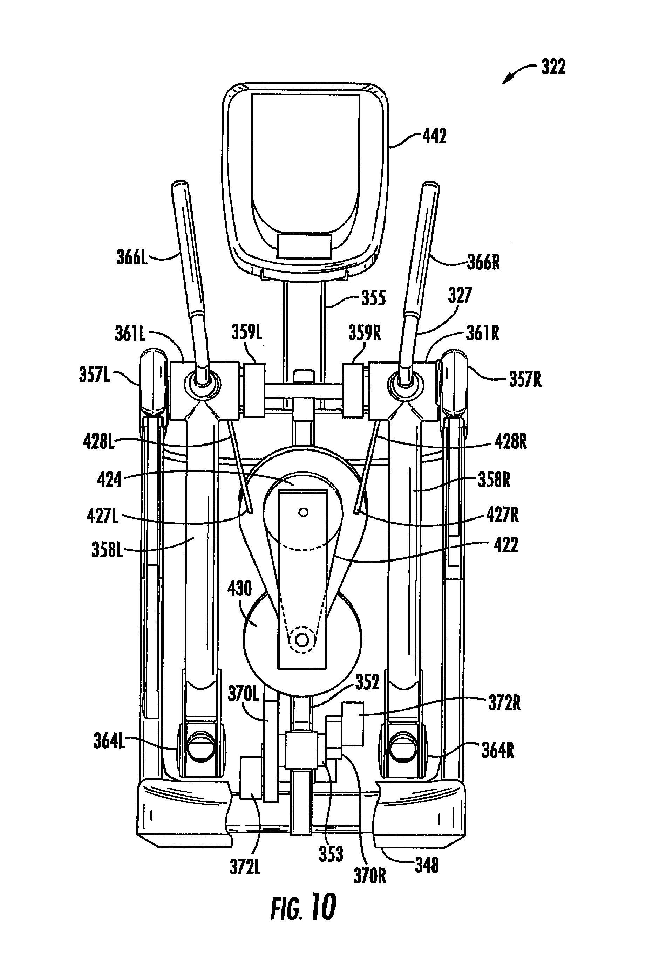

Horizontal resistance system 340 comprises a system configured to apply additional resistance to or against horizontal movement of foot support members 360 and footpads 362. Figures 8-10 illustrate horizontal resistance system 340 in more detail. Figure 10 is a rear view of fitness equipment unit 322 with parts removed to reveal a rear view of horizontal resistance system 340. In the example illustrated, horizontal resistance system 340 is attached to the rearward side of front post 352 in an essentially vertical arrangement such that portions of resistance system 340 rotate about one or more horizontal axes. Such arrangement provides a more compact and efficient design of fitness equipment unit 322. In other embodiments, resistance system 340 may be attached to a different side of front post 352 or to another portion of frame 324.

Horizontal resistance system 340 connecting elements 428R, 428L (collectively referred to as connecting elements 428, upper element mounts 426R, 426L (collectively referred to as upper element mounts 426), lower element mounts 427R, 427L (collectively referred to as lower element mounts 427), resistance source 430 and rocker 424.

Connecting elements 428 comprise rigid linkages or rods. Each of connecting elements 428 has an upper end attached to one of upper element mounts 426 and a lower end attached to one of lower element mounts 427 eccentrically located on rocker 424. Element 428R is attached to mounts 426R and 427R. Element 428L is attached to mounts 426L and 427L. Upper element mounts 426 are attached to hubs 361 associated with linkage assemblies 326. Lower element mounts 427 are operably connected to rocker 424. In the example illustrated, mounts 426 and 427 comprise swivel, universal or pivot joints or the like. Linkage assemblies 326 rotate in opposite directions in response to the forces imposed by upon swing arms 327 and foot supports 360 by the person exercising. As one of linkage assemblies 326 rotates in a clockwise direction as viewed from the left side of fitness equipment unit 322, the upper element mount 426 attached to that linkage assembly 326 correspondingly rotates. The rotation raises the vertical position of element mount 426 and creates upward force on and movement of the element 428 attached to the element mount 426. The upward movement of element 428 results in corresponding movement of lower element mount 427. The movement of lower element mount 427 creates movement of rocker 424, which is operably connected to resistance source 430. In other embodiments, mounts 426 may be secured to other portions of linkage assemblies 326.

Rocker 424 and belt 422 operably connect elements 428 to resistance source 430. Rocker 424 is rotationally driven upon movement of elements 428 against the resistance provided by resistance source 430.

Resistance source 430 comprises a mechanism configured to rotate against a selectively adjustable resistance. In one embodiment, resistance source 430 comprises a metal plate and one or more magnets forming an Eddy brake. In one embodiment, the one or more magnets comprise electromagnets, allowing the strength of the magnetic force to be selectively adjusted to control and vary the resistance applied against the rotation of hubs 361 of linkage assemblies 326. In another embodiment, resistance source 430 may comprise an electric generator. In still another embodiment, resistance source 430 may comprise two surfaces in frictional contact with one another so as to generate resistance against rotation of hubs 361. In another embodiment, air brakes may be utilized. In still other embodiments, other brakes or resistance mechanisms may be utilized. In one embodiment, the resistance applied by horizontal resistance source 430 may be selectively adjusted by a person using fitness equipment unit 322. In one embodiment, the resistance may be adjusted in response to control signals generated by controller 446 associated with interface panel 442 in response to input from a person exercising or in response to a stored exercise routine or workout. In still other embodiments, horizontal resistance system 340 may be omitted.

Interface panel 342 comprises a mechanism facilitating interface between fitness equipment unit 322 and a person exercising. As schematically shown by Figure 4, interface panel 342 comprises display 32, identity input 34, selector input 36, payment input 38, control instructions 40, subscription database 42 and controller 44 (each of which is described above with respect to Figure 1).

During use of fitness equipment unit 322, a person mounts platform 348 while generally grasping side arms 356. While continuing to grasp side arms 356, a person then mounts foot pads 362. The person exercising then inputs via inputs 440 desired workout or exercise routine or selects a pre-stored workout or exercise routine. In response to such inputs, controller 446 may generate control signals adjusting the amount of resistance applied by resistance sources 392 and 430. In addition, controller 446 may generate control signals causing powered actuator 416 to reposition front end flexible element mounts 398 to adjust the step height. During the exercise routine, person exercising may decide to adjust his or her stride or the path of his or her stride. This is achieved by the person simply applying a different force to footpad 362 and linkage assemblies 326. In addition, the person exercising may decide to increase or decrease the step height. To do this, person may simply enter a change using input 440, wherein controller 446 generates control signals causing actuator 416 to reposition adjustment member 414 to adjust the step height. As noted above, this adjustment may be made on the fly during exercise. In other embodiments, controller 446 may automatically adjust the resistance applied by one or both of resistance sources 392 and 430 as well as the step height controlled by step height adjustment mechanism 338 in accordance with stored exercise routine or workout. Such changes may be made based upon the lapse of time from the beginning of the workout, based upon time remaining in the workout, based upon sensed biometrics of the person exercising or based upon predetermined speed, force or motion path objectives or targets being met or not being met. Because fitness equipment unit 322 enables the maximum step height or maximum step depth to be automatically adjusted by controller 446 or to be adjusted by a person during exercise, fitness equipment unit 322 provides more flexible or versatile exercise options and a more enjoyable workout.

As described above, interface panel 342 (shown in Figure 1) includes control instructions 40, subscription database 42 and controller 44. As discussed above with respect to Figure 1, control instructions 40 comprise instructions or control logic embodied as firmware or software in one or more memory structures. Control instructions 40 provide instructions for controller 44. Control instructions 40 comprise different blocks, modules or sets of control programming or routines for the operation of fitness equipment unit 322. In the example illustrated, control instructions 40 comprises base mode instructions 50 and optional or supplemental feature instructions 52A, 52B, 52C, 52D, 52E (shown in Figure 1).

Base mode instructions 50 comprise computer readable programming or instructions for controller 44 so as to control the operation of fitness equipment unit 322 in a default or baseline mode of operation. The baseline mode of operation is a minimum are default operation characteristics for the particular fitness equipment unit 22: the minimum number of paths or ranges of motion, the minimum visual presentations to be presented on display 32 and generally the minimum overall user experience. In one embodiment, absent a subscription plan with any additional supplemental features, controller 44 operates fitness equipment unit 22A or 22B in the base mode. In another embodiment, a baseline subscription plan may include the base mode of operation operating under base mode instructions 50, but not any additional supplemental features which would be provided by supplemental feature instructions 52.

Supplemental feature instructions 52 comprise computer readable programming or instructions so as to control the operation of fitness equipment units 22 with one or more additional supplemental features. Such supplemental features may either replace the base mode features with enhanced features or may add to or build upon the features provided by the base mode of operation. According to one embodiment, such supplemental features are purchasable by the health fitness facility member or user as part of a prescription plan upgrade. The supplemental features may upgrade the operation of display 32, increasing the available content or interactive features or the overall visual presentation quantity presented by controller 44 on display 32 or may upgrade the operation of force receiving members (footpad 326 and their associated linkages), providing a greater range of motion, additional paths, additional or alternative resistance options and the like. In one example embodiment, base mode 50 may comprise a setting wherein height adjustment member 414 (shown in Figure 7) is stationary or fixed such that height adjustment is not offered, wherein optional or upgradable supplemental feature 52A allows adjustment of the positioning of just member 414 such that height adjustments may be made. In another embodiment, supplemental features may include additional adjustment ranges for height. In some embodiments, supplemental features may include different selectable levels or types of resistance against vertical movement, horizontal movement or both. In some embodiments, other optional features may be provided in control instructions 40.

Subscription database 42 comprises a database of current subscription plans 54A, 54B, 54C, 54D, 54E (collectively referred to as subscription plans 54 and shown in Figure 1) for different potential users of the associated fitness equipment unit 322. In one embodiment, subscription database 42 may additionally include a database of different available subscription plans or features that may be purchased along with their associated prices (schematically represented by subscription plan 56). Each subscription plan may include access to or make available the base mode and one or more supplemental features as provided by base mode instruction 50 and supplemental feature instructions 52 under the control of controller 44.

In another embodiment, the prepurchased quantity may be defined in terms of a metric on the fitness equipment unit itself. For example, each subscription plan 54, 56 may include a prepurchased number of miles. In yet other embodiments, the subscription plan 54, 56 may include a pre-purchased quantity relating to wear of the fitness equipment unit 322. In such a manner, the cost of prescription plan could be directly tied to maintenance, repair or replacement of the fitness equipment unit being used.

In yet another embodiment, the subscription plan 54, 56 may include a pre-purchased or predefined universal time period or range for which the particular fitness equipment unit 322 is available for use. For example, the subscription plan may provide user with access to the particular fitness equipment unit on particular days of the week, such as Monday, Wednesday and Friday, only on weekends, during particular hours of each day (8 AM to 11 AM), or during particular hours on selected days (8 AM to 11 AM on Monday Wednesday and Friday, 2 PM to 3 PM on Saturday and 8 PM to 10 PM on Sunday). With such subscription plans 54, 56, prime times or more busy times for a particular fitness equipment unit 22A, 22B may have a higher subscription price while less prime times or less busy times for fitness equipment unit 322 may be provided with a lower subscription price. As a result, available time for use of fitness equipment unit 322 may be allotted at different prices to different users to accommodate different budgets or price points for different users while the same time maximizing income from the fitness equipment unit 322.

Subscription database 42 is stored in a memory structure embodied in fitness equipment unit 322. In other embodiments where similar subscription databases are maintained elsewhere, such as subscription manager 24, subscription database 42 may be omitted as part of the particular fitness equipment unit 322. Although description database 42 is illustrated as including or storing current subscription plans for six users, U1-U6, subscription plans may be enlarged or reduced in size in other embodiments.

Controller 44 comprise one or more processing units provided as part of fitness equipment unit 322 configured to follow instructions, contained in control instructions 40 and based in part upon the current subscription plan for the current member exercising on fitness equipment unit 322, to control the operation of force receiving members 30 and display 32.

In operation according to one embodiment, controller 44 awaits receipt of input or information from identification input 34 identifying a member of the fitness facility or person requesting to use fitness equipment unit 322. Upon receipt of the identification input, controller 44 accesses subscription database 42 and retrieves the current or active subscription plan 54 associated with the particular user. In some embodiments, a "user" may comprise an individual person. In other embodiments, a "user" may comprise an organization or a collective group of individuals who may have purchased a common subscription plan together as a group possibly at a reduced price.

Once the appropriate subscription plan for the particular user that was identified by the identifying input received from identification input 34 has been retrieved by controller 44, controller 44 generate control signals causing display 32 to present a paid subscription status of the user that identifies those features or options currently available in the current subscription plan. Controller 44 follows by awaiting input from selector input 36 identifying which of the available supplemental features that the individual exercising wishes to use during the exercise session. Upon receiving the user's selection, controller 44 accesses the associated control instructions 50 and/or 52 containing instructions 40. Controller 44 generate control signals controlling one or more of display 32, step height adjustment mechanism 338 of the various variable and adjustable resistance sources based upon the selection.

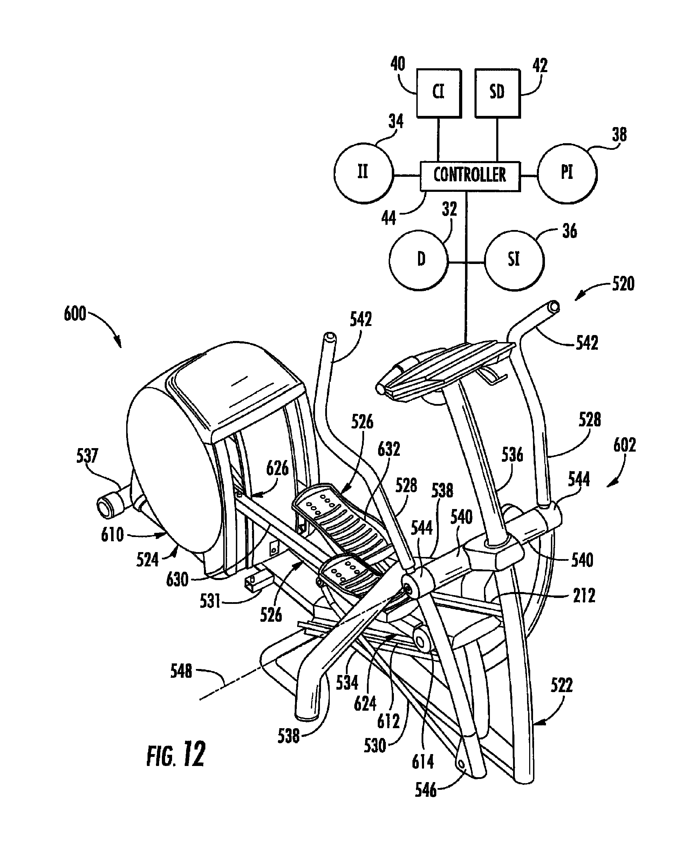

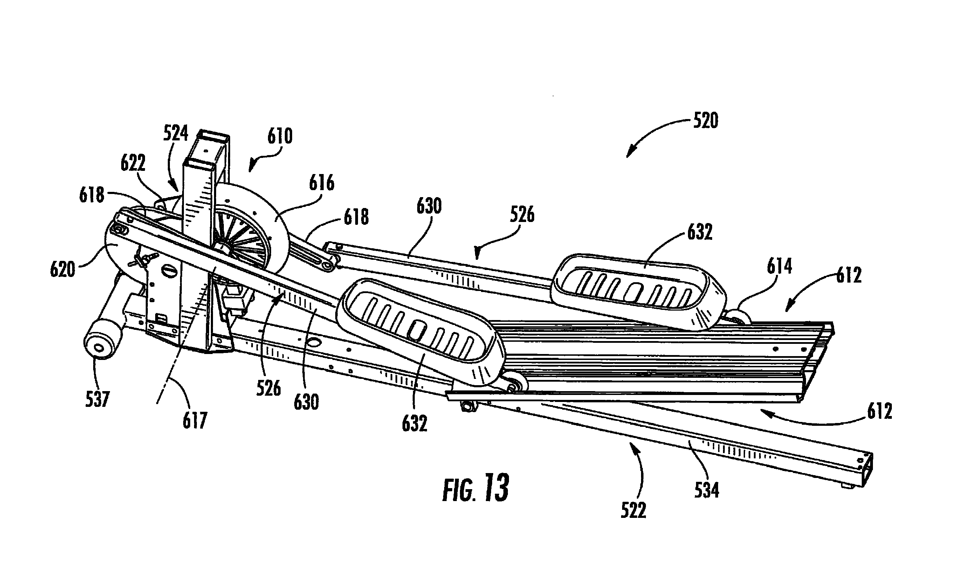

Figures 12 and 13 illustrate fitness equipment unit 520, another example embodiment of FEU 22A or 22B. Fitness Equipment unit 520 includes frame 522, interface panel 523, guide 524, foot links 526, swing arms 528, connection linkages 530 and interface panel 532. Frame 522 comprises one or more structures configured to support the remaining structures are components of exercise device 520 relative to a wall or floor. In the particular example illustrated, frame 522 includes a generally horizontal portion 534, a vertical portion 536, stabilizer portions 537, 538 and swing arm supports 540. Horizontal portion 534 extends along a floor or other support surface while vertical portion 536 extends upwardly from horizontal portion 534. Horizontal portion 534 supports guide 524 while portion 536 supports swing arms 528 and interface panel 532.

Stabilizer portions 537, 538 transversely extend outwardly from horizontal portion 534 to stabilize and support horizontal portion 534. In the example illustrated, stabilizer portion 537 is located at a rear 600 of frame 522 while stabilizer portion 538 is proximate a front 602 of frame 522. In other embodiments, stabilizer portions 537, 538 may have other configurations, may be provided in other locations along frame 522 or may be omitted.

Swing arm supports 540 transversely project from vertical portion 536 proximate to front 602 of frame 522. Swing arm supports 540 pivotably support swing arms 528 for pivotable or rotational movement about axis 548. In particular embodiments, swing arm supports 540 may apply a selected and controlled varying resistance to pivotal movement of swing arms 528. In other embodiments, this feature may be omitted.

Guide 524 comprises an arrangement of one or more structures or one or more mechanisms configured to facilitate movement of foot links 526 relative to frame 522 in one or more paths or manners. Guide 524 controls movement of foot links 526 such that motion or movement of foot links 526 has a reciprocating component. In the embodiment illustrated, guide 524 is configured such that foot links 526 reciprocate in an alternating fashion with respect to one another generally towards and away from interface panel 532 in forward and rearward directions. In the example illustrated, guide 524 is configured such that rearward portions of foot links 526 are constrained to move in an orbital path such that the overall motion of foot links 526 is elliptical.

As shown in more detail by Figure 13, guide 524 includes orbital mechanism 610, guide tracks 612 and engagement rollers 614. Orbital mechanism 610 comprises a mechanism operably connected to rearward portions of foot links 526 and configured so as to constrain movement of rear portions of foot links 526 in an orbital path. In the embodiment illustrated, orbital mechanism 610 comprises a flywheel 616 rotationally supported about a central axis 617 and a pair of crank arms 618, wherein one of the crank arms 616, 618 has a first end rotationally supported about the central axis 617 and a second end rotationally connected to one of foot links 526 and wherein the other of the crank arms 616, 618 has a first end rotationally supported about the central axis and a second end rotationally connected to the other of foot links 526. An example orbital mechanism 610 is described in co-pending US Patent Application Serial No. 11/054,376, published on August 24, 2006 as publication US 2006/0189445, the full disclosure of which is hereby incorporated by reference.

As further shown by Figure 13, in the example embodiment illustrated, orbital mechanism 610 additionally includes resistance supply 620. Resistance supply 620 is operably coupled to flywheel 616 via a belt or pulley 622. Resistance supply 620 provides resistance against the rotation a flywheel 616 about axis 617. In the example illustrated resistance supply 620 comprises a friction brake assembly configured to be adjusted or set at different levels of friction, allowing a user to select a degree a resistance that he or she must overcome during exercise. In other embodiments, resistance supply 620 may have other configurations or may be omitted.

Guide tracks 612 comprise elongate surfaces proximate a forward end of foot links 526 and configured to guide and direct reciprocal movement of a forward end of foot links 526. In the embodiment illustrated down a guide tracks 612 are inclined. For example, in one embodiment, guide tracks 612 are inclined at approximately 30 degrees. Guide tracks 612 receive engagement rollers 614.

Engagement rollers 614 comprise rollers rotationally supported at forward portion of foot links 526. Engagement rollers 614 are configured to roll and move along their respective guide tracks 612. Guide tracks 612 and engagement rollers 614 cooperate with one another to retain engagement rollers 614 relative to guide tracks 612 in a reliable, compact and less complex manner.

Each foot link 526 comprises one or more structures configured to engage a person's leg or foot such that movement of the person's leg or foot causes movement of foot link 526. In the embodiment illustrated, each foot link 526 includes a support 630 and a foot rest 632. Support 630 comprises an elongate bar, rod or otherwise rigid structure having a forward end 624 supporting engagement roller 614 and a rear end 626 connected to orbital mechanism 610. Each foot rest 632 comprises a pedal or other surface upon which a person may place his or her foot to transfer force to foot link 526. In one embodiment, foot rests 632 are configured to form toe straps and/or toe and heel cups which aid in forward motion recovery at the end of a rearward or forward striding motion of a user's foot.

As shown by Figure 12, swing arms 528 comprise one or more structures configured to be gripped by a person's hand and to be reciprocated to exercise a person's arms and upper body. Each swing arm 528 includes a gripping portion 542, an intermediate portion 544 pivotably connected to support 540 of frame 522 and an end portion 546 pivotably connected to connection link 530. Gripping portion 542 comprises that portion of swing arm 528 configured to be grasped or gripped by a person's hand. Intermediate portion 544 facilitates pivotal movement of swing arm 528 about the substantially horizontal axis 548. Although swing arms 528 are illustrated as being bowed, in other embodiments, swing arms 528 may have other shapes, relative dimensions and configurations.

Each connection link 530 comprises one or more segments or links configured to connect foot link 526 and swing arm 528 such that movement of foot link 526 and swing arm 528 is coordinated. In one embodiment, connection link 530 is configured and appropriately connected to foot link 526 and swing arm 528 such that when foot link 526 is moving forwardly (towards interface panel 523), swing arm 528 is moving rearwardly. When foot link 526 is moving rearwardly, swing arm 528 is moving forwardly. In other words, when foot link 526 is in a forward most position, the connection between connection link 530 and swing arm 528 is on an opposite side of axis 548 as gripping portion 542. In some embodiments, connection links 530 may be disconnected from either foot links 526 or swing arms 528. In still other embodiments, connection links 530 may be omitted, wherein swing arms 528 swing independent of foot links 526 or are stationary.

Interface panel 523 comprises an electronic device configured to interface with a person using exercise device 520. In one embodiment, interface 123 facilitates input of instructions or commands by the person or from an external source. Such commands may be used to set or establish levels of resistance, speed or other settings to vary or control work out parameters. In one embodiment, interface 123 may additionally or alternatively be configured to provide the person with information or feedback regarding the current workout. Interface 523 is additionally configured to provide a person using exercise device 520 with information regarding exercise goals, past workouts, recommended settings or entertainment information, such as news, videos or music. In one embodiment, interface panel 523 may be configured to communicate with other external electronic devices, such as other computers, servers or portable devices in a wired or wireless fashion.

Similar to interface panel 342, interface panel 523 (shown in Figure 12) includes control instructions 40, subscription database 42 and controller 44. As discussed above with respect to Figure 1, control instructions 40 comprise instructions or control logic embodied as firmware or software in one or more memory structures. Control instructions 40 provide instructions for controller 44. Control instructions 40 comprise different blocks, modules or sets of control programming or routines for the operation of fitness equipment unit 322. In the example illustrated, control instructions 40 comprises base mode instructions 50 and optional or supplemental feature instructions 52A, 52B, 52C, 52D, 52E (shown in Figure 1).

Base mode instructions 50 comprise computer readable programming or instructions for controller 44 so as to control the operation of fitness equipment unit 322 in a default or baseline mode of operation. The baseline mode of operation is a minimum are default operation characteristics for the particular fitness equipment unit 520: the minimum number of paths or ranges of motion, the minimum visual presentations to be presented on display 32 and generally the minimum overall user experience. In one embodiment, absent a subscription plan with any additional supplemental features, controller 44 operates fitness equipment unit 22A or 22B in the base mode. In another embodiment, a baseline subscription plan may include the base mode of operation operating under base mode instructions 50, but not any additional supplemental features which would be provided by supplemental feature instructions 52.

Supplemental feature instructions 52 comprise computer readable programming or instructions so as to control the operation of fitness equipment units 22 with one or more additional supplemental features. Such supplemental features may either replace the base mode features with enhanced features or may add to or build upon the features provided by the base mode of operation. According to one embodiment, such supplemental features are purchasable by the health fitness facility member or user as part of a prescription plan upgrade. The supplemental features may upgrade the operation of display 32, increasing the available content or interactive features or the overall visual presentation quantity presented by controller 44 on display 32 (as described above) or may upgrade the operation of force receiving members (footpad 232 and their associated linkages), providing a greater range of motion, additional or alternative resistance options and the like. In some embodiments, supplemental features may include different selectable levels or types of resistance against vertical movement, horizontal movement or both. In some embodiments, other optional features may be provided in control instructions 40.

In one embodiment, each subscription plan 54, 56 may additionally include a pre-purchased quantity relating to the use of fitness equipment unit 520. For example, each subscription plan 54, 56 may additionally include a prepurchased number of hours that the subscriber may use a particular fitness equipment unit 520. The hours quantity may be a total number of hours which is gradually consumed by a subscriber during use of the fitness pivot unit or may be a total number of hours during a predefined time period (e.g., 5 hours per week; 15 hours per month; 5 hours per calendar week; 15 hours per calendar year).

In yet another embodiment, the subscription plan 54, 56 may include a pre-purchased or predefined universal time period or range for which the particular fitness equipment unit 520 is available for use. For example, the subscription plan may provide user with access to the particular fitness equipment unit on particular days of the week, such as Monday, Wednesday and Friday, only on weekends, during particular hours of each day (8 AM to 11 AM), or during particular hours on selected days (8 AM to 11 AM on Monday Wednesday and Friday, 2 PM to 3 PM on Saturday and 8 PM to 10 PM on Sunday). With such subscription plans 54, 56, prime times or more busy times for a particular fitness equipment unit 520 may have a higher subscription price while less prime times or less busy times for fitness equipment unit 520 may be provided with a lower subscription price. As a result, available time for use of fitness equipment unit 520 may be allotted at different prices to different users to accommodate different budgets or price points for different users while the same time maximizing income from the fitness equipment unit 322.

Subscription database 42 is stored in a memory structure embodied in fitness equipment unit 322. In other embodiments where similar subscription databases are maintained elsewhere, such as subscription manager 24, subscription database 42 may be omitted as part of the particular fitness equipment unit 520. Although description database 42 is illustrated as including or storing current subscription plans for six users, U1-U6, subscription plans may be enlarged or reduced in size in other embodiments.

Controller 44 comprise one or more processing units provided as part of fitness equipment unit 322 configured to follow instructions, contained in control instructions 40 and based in part upon the current subscription plan for the current member exercising on fitness equipment unit 322, to control the operation of force receiving members 232 and display 32.

Once the appropriate subscription plan for the particular user that was identified by the identifying input received from identification input 34 has been retrieved by controller 44, controller 44 generate control signals causing display 32 to present a paid subscription status of the user that identifies those features or options currently available in the current subscription plan. Controller 44 follows by awaiting input from selector input 36 identifying which of the available supplemental features that the individual exercising wishes to use during the exercise session. Upon receiving the user's selection, controller 44 accesses the associated control instructions 50 and/or 52 containing instructions 40. Controller 44 generates control signals controlling display 32 and adjusts resistance sources based upon the selection.

Although the present disclosure has been described with reference to example embodiments, workers skilled in the art will recognize that changes may be made in form and detail without departing from the spirit and scope of the claimed subject matter. For example, although different example embodiments may have been described as including one or more features providing one or more benefits, it is contemplated that the described features may be interchanged with one another or alternatively be combined with one another in the described example embodiments or in other alternative embodiments. Because the technology of the present disclosure is relatively complex, not all changes in the technology are foreseeable. The present disclosure described with reference to the example embodiments and set forth in the following claims is manifestly intended to be as broad as possible. For example, unless specifically otherwise noted, the claims reciting a single particular element also encompass a plurality of such particular elements.

## Claims

1. A fitness equipment subscription system comprising:
a fitness equipment unit operable in a base mode with one or more selectable optional supplemental features;
one or more inputs associated with the fitness equipment unit that receives input identifying a user using the fitness equipment unit; and
a controller in communication with the input and the fitness equipment unit, wherein the controller is configured to at least one of make available the one or more optional features based upon the identity of the user and a subscription plan associated with the user, and limit use of the fitness equipment unit based on a subscription plan associated with the user.

2. The system of claim 1, wherein the subscription plan is purchased by the user.

3. The system of claim 1 or 2, wherein the fitness equipment unit includes a display.

4. The system of claim 3, wherein the controller generates control signals causing the one or more selectable features to be presented on the display, and optionally wherein the base mode comprises a first set of visual presentations on the display and wherein the one or more optional supplemental features comprise a second set of visual presentations on the display.

5. The system of claim 3 or 4, wherein the controller is configured to cause the display to present a paid subscription status of the user that identifies purchases of optional supplemental features, and optionally wherein the controller is configured to cause the display to present offers for additional supplemental features not currently in the subscription plan of the user.

6. The system of any preceding claim, wherein the one or more inputs are configured to receive payment authorization from the user for the purchase of additional supplemental features, and/or wherein the one or more inputs are configured to receive a selection of a feature contained in the subscription plan of the user.

7. The system of any preceding claim, wherein the base mode comprises a first set of one or more exercise motions and wherein the one or more optional supplemental features comprises a second set of one or more exercise paths of motion.

8. The system of any preceding claim, wherein the fitness equipment unit defines a first fitness equipment unit and the system comprises a second fitness equipment unit having associated one or more inputs, and wherein the controller is in communication with a second fitness equipment unit.

9. The system of claim 8, wherein the second fitness equipment unit is operable in the base mode and the one or more selectable optional supplemental features, and the controller is configured to make available the one or more optional features on the second fitness equipment unit based upon an identity of a second user and a second subscription plan associated with the second user.

10. The system of claim 8, wherein the first fitness equipment unit is operable in a first base mode and one or more first selectable optional supplemental features, and the second fitness equipment unit is operable in a second base mode different than the first base mode and one or more second selectable optional supplemental features, and the controller is configured to make available the one or more second optional features on the second fitness equipment unit based upon an identity of a second user and a second subscription plan associated with the second user.

11. The system of any preceding claim, wherein the one or more optional features comprises forwarding at least a media resumption point from the fitness equipment unit to an external media player.

12. The system of any preceding claim, wherein the subscription plan associated with the user identifies a quantity of use of the fitness equipment unit available to the user, and/or wherein the subscription plan associated with the user identifies a universal time period available to the user for using the fitness equipment unit.

13. A method comprising:
receiving an identification of a user at a fitness equipment unit;
retrieving a current subscription plan for the user based upon the identification;
making features for the fitness equipment unit in the current subscription plan of the user available for use by the user on the fitness equipment unit.

14. The method of claim 13, wherein the current subscription plan is retrieved from a database of a plurality of subscription plans for a plurality of potential users of the fitness equipment unit, and /or wherein the subscription plan comprises features for a plurality of different fitness equipment units for each of the plurality of potential users.

15. The method of claim 13 or 14 further comprising:
making features in the current subscription plan available for selection by the user at the fitness equipment unit;
receiving a selection of one or more of the features in the current subscription plan for the user;
controlling the fitness equipment unit to use the one or more features based on the selection.
